# EUROPEAN PATENT SPECIFICATION

(11) **EP 1 414 813 B1**
(45) Date of publication and mention of the grant of the patent: **13.09.2006**
(21) Application number: 02763208.2
(22) Date of filing: 21.06.2002
(51) Int. Cl.: C07D 323/02, C07D 493/10, C07D 405/14, C07D 405/08, C07D 419/08, C07D 521/00, A61P 33/06

(54) **1,2,4-TRIOXOLANE ANTIMALARIALS**
1,2,4 TRIOXOLAN-ANTIMALARIAMITTEL
ANTIPALUDIQUES A BASE DE 1,2,4-TRIOXOLANE

(30) Priority: 21.06.2001 US 886666
(43) Date of publication of application: 06.05.2004
(73) Proprietor: Medicines for Malaria Venture, 1215 Geneva 15 (CH)
(72) Inventor: VENNERSTROM, J., University of Nebraska Med. Ctr., Omaha, NE 68198-6025 (US); CHOLLET, Jacques, Pharma Research Department, CH-4002 Basel (CH); DONG, Y., University of Nebraska Medical Center, Omaha, NE 68198-6025 (US); MATILE, Hugues, Pharma Research Department, CH-4002 Basel (CH); PADMANILAYAM, M., University of Nebraska Med. Ctr., Omaha, NE 68198-6025 (US); TANG, Y., University of Nebraska Medical Center, Omaha, NE 68198-6025 (US); CHARMAN, William N., Victorian College of Pharmacy, Parkville, VIC 3052 (AU)
(74) Representative: Tomkinson, Alexandra
(86) International application number: PCT/US2002/019767
(87) International publication number: WO 2003/000676

(56) References cited:
- HELMUT KEUL: "Über Konstitution und Entstehung der Ozonide von Bis-adamantyliden und Bis-bicyclo[3.3.1]non-9-yliden " CHEMISCHE BERICHTE, vol. 108, no. 4, 1975, pages 1207-1217, XP002217805
- T. TABUCHI, M. NOJIMA: "Ozonolysis of vinyl ethers in the presence of alpha-diketones and alpha-keto esters" J. ORG. CHEM., vol. 56, 1991, pages 6591-6595, XP001117555
- P. H. DUSSAULT, H.-J. LEE, X. LIU: "Selectivity in Lewis acid-mediated fragmentations of peroxides and ozonides; application to the synthesis of alkenes, homoallylethers, and 1,2-dioxolanes" PERKIN TRANS. , vol. 1, 2000, pages 3006-3013, XP001117556
- GRIESBAUM K ET AL: "Diozonides from Coozonolyses of Suitable O-Methyl Oximes and Ketones" TETRAHEDRON, ELSEVIER SCIENCE PUBLISHERS, AMSTERDAM, NL, vol. 53, no. 15, 14 April 1997 (1997-04-14), pages 5463-5470, XP004105588 ISSN: 0040-4020
- MESHNICK S R ET AL: "ARTEMISININ AND THE ANTIMALARIAL ENDOPEROXIDES: FROM HERBAL REMEDY TO TARGETED CHEMOTHERAPY" MICROBIOLOGICAL REVIEWS, AMERICAN SOCIETY FOR MICROBIOLOGY, WASHINGTON, DC, US, vol. 60, no. 2, 1 June 1996 (1996-06-01), pages 301-315, XP002052313 ISSN: 0146-0749 cited in the application
- JEFFORD ET AL: "Peroxidic Antimalarials" ADVANCES IN DRUG RESEARCH, ACADEMIC PRESS, LONDON, GB, vol. 29, 1997, pages 271-325, XP002119844 ISSN: 0065-2490 cited in the application

## Description

### FIELD OF THE INVENTION

This invention relates to compositions and methods for treating malaria. Specifically, this invention relates to pharmaceutical compositions including spiro and dispiro trioxolanes, and methods of their use and manufacture.

### BACKGROUND OF THE INVENTION

Malaria is an acute and often chronic infectious disease resulting from the presence of protozoan parasites within red blood cells. Caused by single-celled parasites of the genus *Plasmodium,* malaria is transmitted from person to person by the bite of female mosquitos.

Although once prevalent in North America and other temperate regions of the world, today malaria occurs mostly in tropical and subtropic countries. Each year, between 400 million and 600 million people contract the disease, and 1.5 million to 2.7 million die of the disease.

Four species of *Plasmodium* protozoan parasites are generally responsible for malaria, including *Plasmodium vivax, Plasmodium falciparum, Plasmodium malariae,* and *Plasmodium ovale.* Of the four, *Plasmodium falciparum* is the most dangerous, accounting for half of all clinical cases of malaria and 90% of deaths from the disease.

The transmission of malaria begins when a female mosquito bites a human already infected with the malaria parasite. When the infected mosquito bites another human, sporozoites in the mosquito's saliva are transferred into the blood, which then travel to the liver. In the liver, the sporozoites divide rapidly, then enter the bloodstream where they invade red blood cells. Inside these blood cells, the merozoites multiply rapidly until they cause the red blood cells to burst, releasing into the blood stream a new generation of merozoites that then infect other red blood cells.

The symptoms associated with malaria are generally associated with the bursting of the red blood cells. The destruction of the red blood cells spills wastes, toxin, and other debris into the blood. This in turn causes an intense fever that can leave the infected individual exhausted and bedridden. More severe symptoms associated with repeat infections and/or infection by *Plasmodium falciparum* include anemia, severe headaches, convulsions, delirium and, in some instances, death.

The treatment of malaria has been especially difficult due to the ability of malaria parasites to develop resistance to drugs. Quinine, an antimalarial compound that is extracted from the bark of the South American cinchona tree, is one of the oldest and most effective pharmaceuticals in existence. The downside to quinine is that it is short-acting, and fails to prevent disease relapses. Further, quinine is associated with side effects ranging from dizziness to deafness.

Chloroquine is a synthetic chemical similar to quinine. It became the drug of choice for malaria when it was developed in the 1940s due to its effectiveness, ease of manufacture, and general lack of side effects. However, in the last few decades, malaria parasites in many areas of the world have become resistant to chloroquine.

Mefloquine is another synthetic analog of quinine that has been used in the treatment of malaria. Malaria parasites have also developed resistance to mefloquine, however. Mefloquine is also associated with undesirable central nervous side effects in some patients, including hallucinations and vivid nightmares.

Antifolate drugs are effective against malaria parasites by inhibiting their reproduction. Although the parasites have also developed a resistance to antifolate drugs, the drugs can still be used effectively in combination with other types of antimalarials. The use of combination therapies in treating malaria has the drawbacks of being inconvenient and expensive, however.

More recent developments in the treatment of malaria have involved the use of the peroxide functional group, as exemplified by the drug artemisinin, which contains a unique 1,2,4-trioxane heterocyclic pharmacophore. The antimalarial action of artemisinin is due to its reaction with the iron in free heme molecules in the malaria parasite with the generation of free radicals leading to cellular destruction.

The discovery of artemisinin (qinghaosu), a naturally occurring endoperoxide sesquiterpene lactone (Meshnick et al., 1996; Vroman et al. 1999; Dhingra et al., 2000) initiated a substantial effort to elucidate its molecular mechanism of action (Jefford, 1997; Cumming et al., 1997) and to identify novel antimalarial peroxides (Dong and Vennerstrom, 2001). Many synthetic 1,2,4-trioxanes, 1,2,4,5-tetraoxanes, and other endoperoxides have been prepared.

Although the clinically useful semisynthetic artemisinin derivatives are rapid acting and potent antimalarial drugs, they have several disadvantages including recrudescence, neurotoxicity, (Wesche et al., 1994) and metabolic instability. (White, 1994). A fair number of these compounds are quite active *in vitro,* but most suffer from low oral activity. (White, 1994; van Agtmael et al., 1999). Although many synthetic antimalarial 1,2,4-trioxanes have since been prepared (Cumming et al., 1996; Jefford, 1997), there exists a need in the art to identify new peroxide antimalarial agents, especially those which are easily synthesized, are devoid of neurotoxicity, and which possess improved pharmacokinetic properties, e.g. improved stability, oral absorption, etc.

Various trioxolanes have been synthesized by various methods. See e.g. Keul, H., Chemische Berichte, vol. 108, no. 4, 1975, pp. 1207-1217; Tabuchi, T. et al., J. Org. Chem., vol. 56, 1991, pp. 6591-6595; Dussault, P.H., Perkin Trans., vol. 1, 2000, pp. 3006-3013; Griesbaum K. et al., Tetrahedron, vol. 53, no. 15, 1997, pp. 5463-5470. However, until now it has not been known in the art that certain types of trioxolanes are especially useful in the prophylaxis and treatment of malaria.

Accordingly, it is a primary objective of the present invention to provide compositions and methods for prophylaxis and treatment of malaria using spiro and dispiro 1,2,4-trioxolanes.

It is a further objective of the present invention to provide a composition and method for prophylaxis and treatment of malaria using spiro and dispiro 1,2,4-trioxolanes that is nontoxic.

It is a further objective of the present invention to provide a composition and method for prophylaxis and treatment of malaria using spiro and dispiro 1,2,4-trioxolanes that is metabolically stable and orally active.

It is yet a further objective of the present invention to provide a composition and method for prophylaxis and cost-effective treatment of malaria using spiro and dispiro 1,2,4-trioxolanes.

It is a further objective of the present invention to provide compositions and methods for prophylaxis and treatment of malaria using spiro and dispiro 1,2,4-trioxolanes that can be used either as stand-alone medicaments or in combination with other agents.

It is still a further objective of the present invention to provide novel intermediates for synthesizing compositions for prophylaxis and treatment of malaria.

The method and means of accomplishing each of the above objectives as well as others will become apparent from the detailed description of the invention which follows hereafter.

### SUMMARY OF THE INVENTION

The invention describes a method and composition for treating malaria with spiro and dispiro 1,2,4-trioxolanes. The trioxolanes of this invention are sterically hindered on one side of the trioxolane heterocycle in order to provide chemical and metabolic stability to the trioxolane ring for better *in vivo* activity. The spiro and dispiro trioxolanes are preferably sterically hindered with an unsubstituted, mono-, di-, or poly-substituted C₅-C₁₂ spiro cycloalkyl group, which is most preferably spiroadamantane. The spiro and dispiro trioxolanes also preferably include a spirocyclohexyl that is preferably functionalized or substituted at the 4-position or a spiropiperidyl ring that is functionalized or substituted at the nitrogen atom. The invention embraces achiral, achiral diastereomers, racemic mixtures, as well as enantiomeric forms of the compounds.

The trioxolanes of this invention possess excellent potency and efficacy against *Plasmodium* parasites, and a low degree of neurotoxicity. In addition, several of the trioxolanes are suitable for both oral and non-oral administration. Moreover, in comparison to artemisinin semisynthetic derivatives, the compounds of this invention are structurally simple, easy and inexpensive to synthesize, and can be used effectively alone or in conjunction with other antimalarials.

### DETAILED DESCRIPTION OF THE PREFERRED EMBODIMENT

The present invention relates to the development of spiro and dispiro 1,2,4-trioxolanes for use in the prophylaxis and treatment of malaria. The present invention is predicated upon the unexpected discovery that trioxolanes that are relatively sterically hindered on at least one side of the trioxolane heterocycle provide metabolic and chemical stability to the trioxolane ring, thereby providing better *in vivo* activity, especially with respect to oral administration.

As used herein the term "prophylaxis-effective amount" refers to a concentration of compound of this invention that is effective in inhibiting or preventing infection and subsequent disease by malarial parasites. Likewise, the term "treatment-effective amount" refers to a concentration of compound that is effective in treating malaria in terms of preventing an increase in the concentration of malarial parasites, decreasing the concentration of malarial parasites, and/or "curing" a malaria infection, i.e. survival for 30 days post-infection.
According to the present invention there is provided a spiro or dispiro 1,2,4-trioxolane having the following structure: 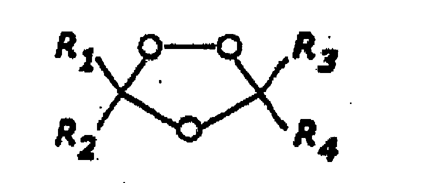 wherein R₁ and R₂ taken together is spiroadamantane and R₃ and R₄ taken together is a spirocyclohexyl ring that is substituted at the 4-position.
The invention describes a method and composition for treating malaria with spiro and dispiro 1,2,4-trioxolanes, their prodrugs and analogues. The trioxolanes of this invention are sterically hindered on one side of the trioxolane heterocycle in order to provide chemical and metabolic stability to the trioxolane ring for better *in vivo* activity. The spiro and dispiro trioxolanes are preferably sterically hindered with an unsubstituted, mono-, di-, or poly-substituted C₅-C₁₂ spiro cycloalkyl group, which is most preferably spiroadamantane. The spiro and dispiro trioxolanes also preferably include a spirocyclohexyl that is preferably functionalized or substituted at the 4-position or a spiropiperidyl ring that is functionalized or substituted at the nitrogen atom. The invention embraces achiral, achiral diastereomers, racemic mixtures, as well as enantiomeric forms of the compounds.

The trioxolanes of this invention possess excellent potency and efficacy against *Plasmodium* parasites, and a low degree of neurotoxicity. In addition, several of the trioxolanes are suitable for both oral and non-oral administration. Moreover, in comparison to artemisinin semisynthetic derivatives, the compounds of this invention are structurally simple, easy and inexpensive to synthesize, and can be used effectively alone or in conjunction with other antimalarials.

### DETAILED DESCRIPTION OF THE PREFERRED EMBODIMENT

The present invention relates to the development of spiro and dispiro 1,2,4-trioxolanes for use in the prophylaxis and treatment of malaria. The present invention is predicated upon the unexpected discovery that trioxolanes that are relatively sterically hindered on at least one side of the trioxolane heterocycle provide metabolic and chemical stability to the trioxolane ring, thereby providing better *in vivo* activity, especially with respect to oral administration.

As used herein the term "prophylaxis-effective amount" refers to a concentration of compound of this invention that is effective in inhibiting or preventing infection and subsequent disease by malarial parasites. Likewise, the term "treatment-effective amount" refers to a concentration of compound that is effective in treating malaria in terms of preventing an increase in the concentration of malarial parasites, decreasing the concentration of malarial parasites, and/or "curing" a malaria infection, i.e. survival for 30 days post-infection.

Tetrasubstituted trioxolanes are relatively stable peroxidic compounds based on literature precedent (Griesbaum et al., 1997a; 1997b). This may be due, in part, to the lack of α-hydrogen atoms. The present inventors have synthesized new compounds in the trioxolane class having both superior antimalarial potency and oral efficacy. Furthermore, the compounds of this invention have low toxicity, and half-lives conducive to treatment of malaria which are believed will permit short-term treatment regimens comparing favorably to other artemisinin-like drugs. These compounds may also be used in malaria prophylaxis.

The tetrasubstituted trioxolanes of this invention have the following general structural formula: 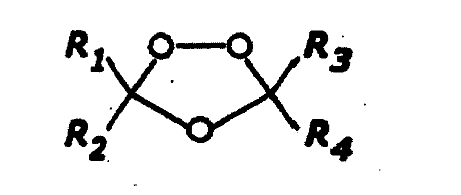 wherein R₁, R₂, R₃, and R₄ represent combinations of ring systems, acyclic systems, and functional groups that provide sufficient steric hindrance about the trioxolane ring in order to give the ring chemical and metabolic stability. R₁, R₂, R₃ and R₄ may be the same or different, and may be a linear or branched alkyl, aryl, or alkaryl group which is optionally substituted. In the alternative, R₁ and R₂ taken together and/or R₃ and R₄ taken together may form an alicyclic group which is optionally interrupted by one or more oxygen, sulfur or nitrogen atoms and which group is optionally substituted. In no event may any of R₁, R₂, R₃ or R₄ be hydrogen.

Preferably, R₁ and R₂ taken together and/or R₃ and R₄ taken together is a mono- or di-substituted C₅-C₁₂ spirocycloalkyl group which is optionally interrupted by one or more oxygen, sulfur, or nitrogen atoms, and which group is optionally substituted.

Most preferably, R₁ and R₂ taken together or R₃ and R₄ is spiroadamantane. It is hypothesized that the sterically demanding adamantane protects the trioxolane ring from premature chemical or metabolic decomposition in situ.

The inventors have further found that in the most preferred compounds of this invention, R₁ and R₂ taken together is adamantane, and R₃ and R₄ taken together is a spirocyclohexyl ring that is functionalized or substituted at the 4-position. The spirocyclohexyl ring may be optionally interrupted by one or more oxygen, sulfur or nitrogen atoms. The functional group may be a linear or branched alkyl, ketone, acid, alcohol, amine, amide, sulfonamide, guanidine, ether, ester, oxime, urea, oxime ether, sulfone, lactone, carbamate, semicarbazone, phenyl, heterocycle, or alicyclic group which is optionally substituted. The functional group is preferably an amide. It has now been unexpectedly found that amide-containing substituents at the 4-position provide antimalarial compounds with good oral absorption, good antimalarial activity, and good pharmacokinetics, i.e. rates of absorption, metabolism, and elimination that are most suitable and advantageous for the prophylaxis and treatment of malaria.

It has also been found that a heteroatom in the spirocyclohexyl ring generally causes the compound to more rapidly metabolize. Thus, for purposes of the pharmaceutical compositions of this invention, such compounds are not preferred.

Other substituents at the 4-position of the spirocyclohexyl ring are also possible that fall within the scope of this invention. The spirocyclohexyl ring may also be substituted at other positions besides the 4-position. For instance, the inventors have synthesized several compounds substituted at the 2-position of the spirocyclohexyl ring that provide excellent antimalarial potency.

Preferred compounds of this invention include an alkyl group connecting the substituent at the 4-position to the spirocyclohexyl ring. The alkyl group is preferably methyl or ethyl, with methyl being most preferred. The alkyl "bridge" group also improves the metabolically stability (i.e. oral activity and pharmacokinetics) of the antimalarial compounds of this invention.

The present inventors have identified two orally active lead dispiro-1,2,4-trioxolanes, OZ03 and OZ05: 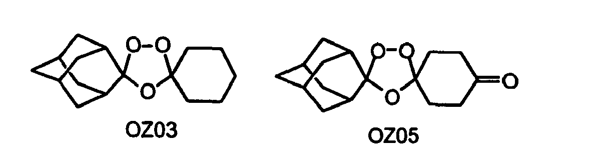 These trioxolanes have IC₅₀ₛ between 1 and 5 ng/ml against *P. falciparum in vitro,* and presumably possess good therapeutic indices as no toxicity is evidence for either compound in a neuroblastoma cell line or at single 640 mg/kg doses in mice in the Rane test. These results contrast with published data (de Almeida Barbosa et al., 1992; 1996) disclosing the weak in vitro antimalarial potency of several tricyclic trioxolanes, the best of which has an IC₅₀ of 2000 ng/ml against *P. falciparum in vitro.*

A notable feature of these trioxolanes in comparison to the artemisinin semisynthetic derivatives is their structural simplicity. A potential advantage of trioxolanes over both trioxanes (Jefford, 1997; Cumming et al., 1997) and tetraoxanes (Vennerstrom et al., 2000) is a more convenient access to structurally diverse, non-symmetrical, and in many cases, achiral compounds.

Below are several dispiro 1,2,4-trioxolanes synthesized in accordance with the teachings of this invention. "OZ" is an internal designation for these compounds that will be used throughout the remainder of the application for convenience:

### OZ Series (OZ01-OZ09)

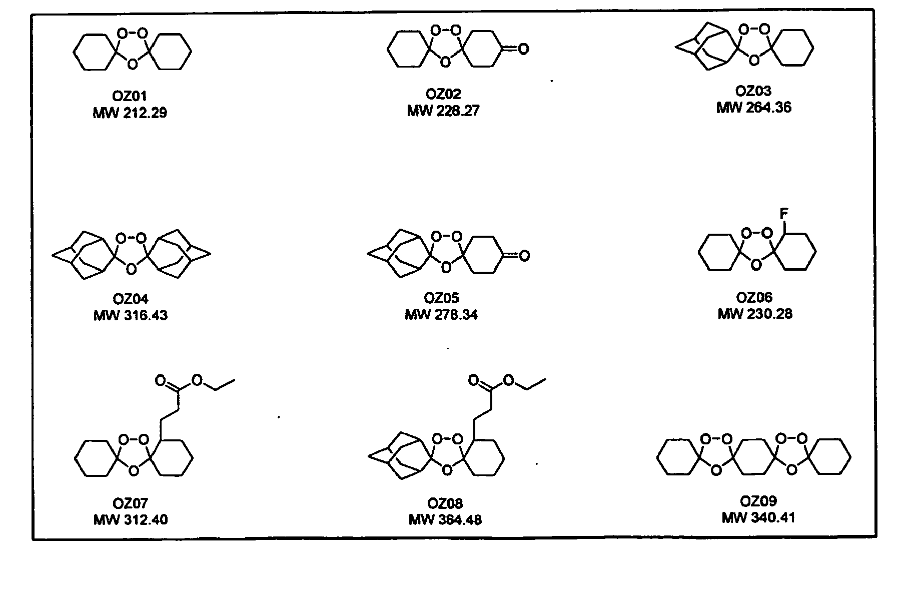

### OZ Series (OZ10-OZ18)

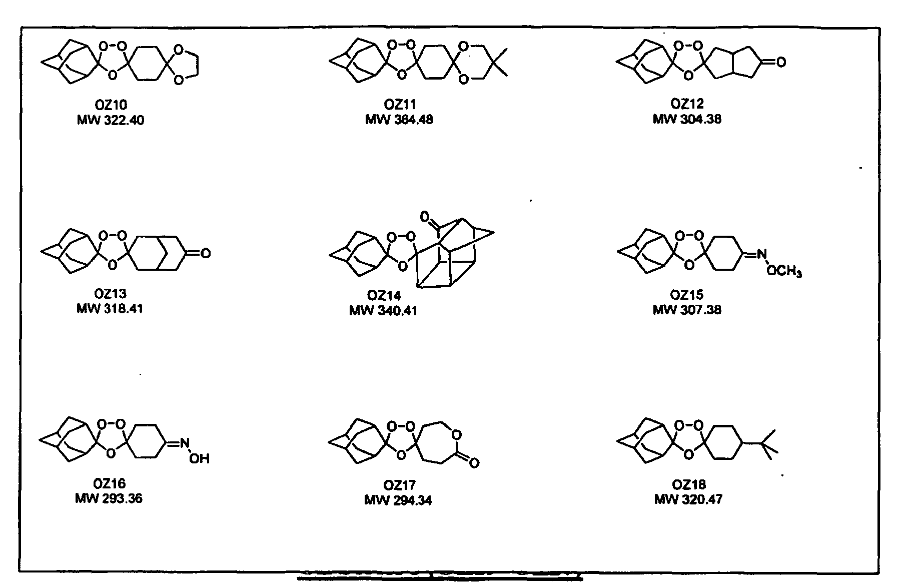

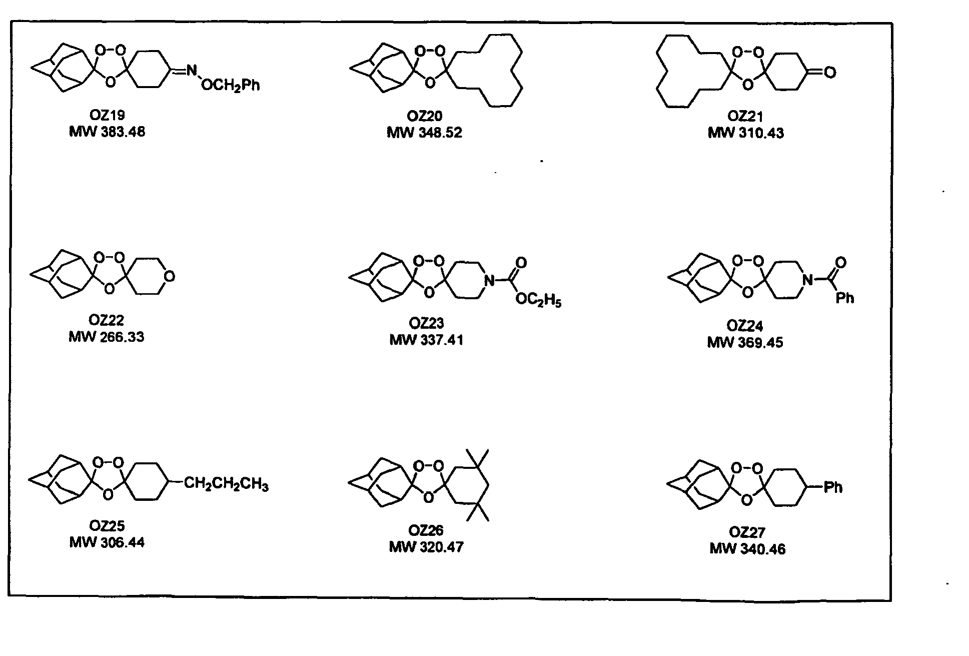

### OZ Series 4 (OZ28-OZ36)

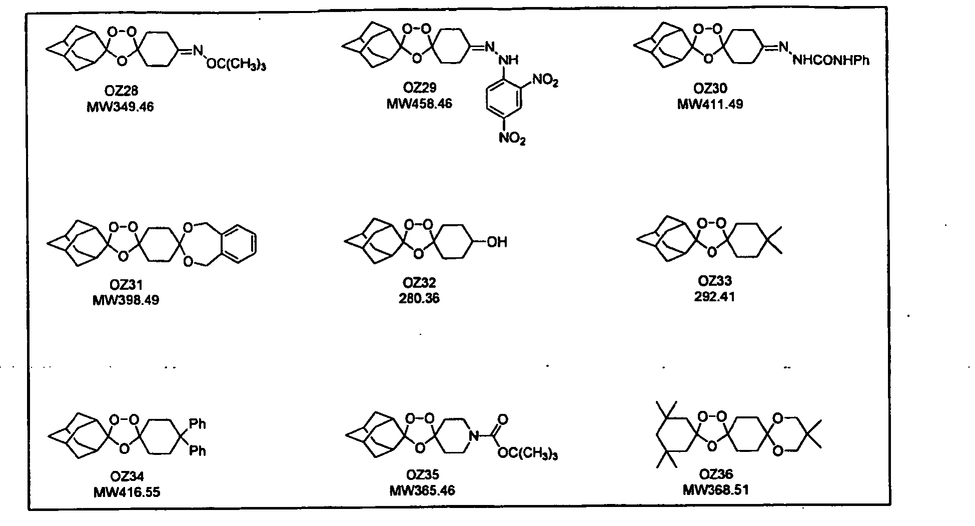

### OZ Series 5 (OZ37-OZ45)

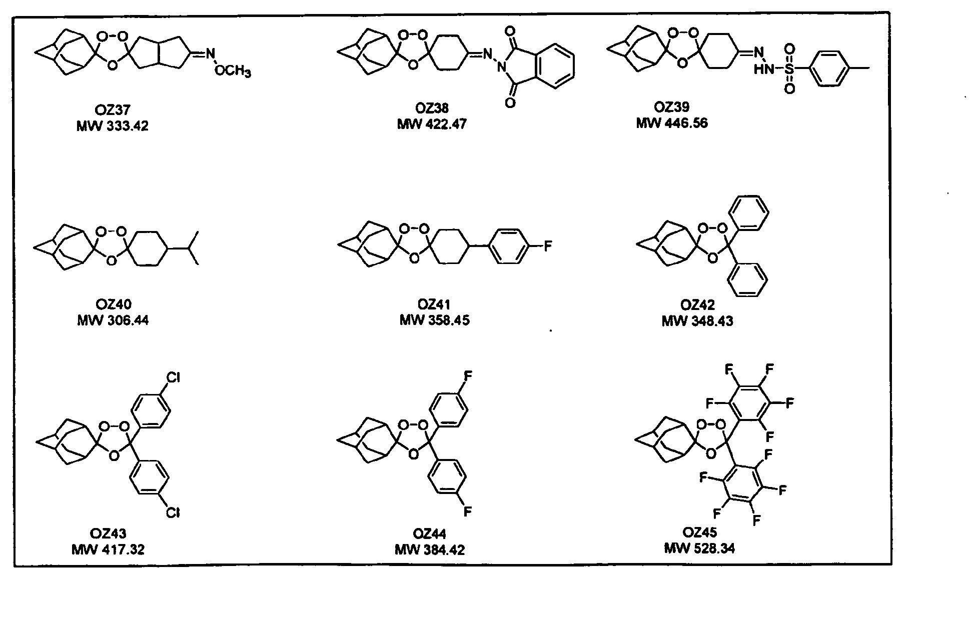

### OZ Series 6 (OZ46-OZ54)

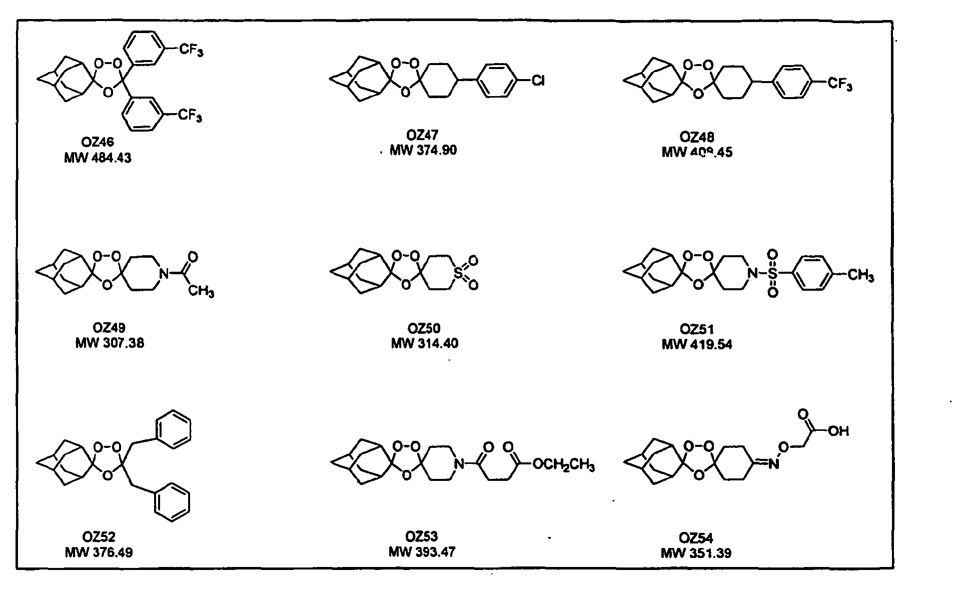

### OZ Series 7 (OZ55-OZ63)

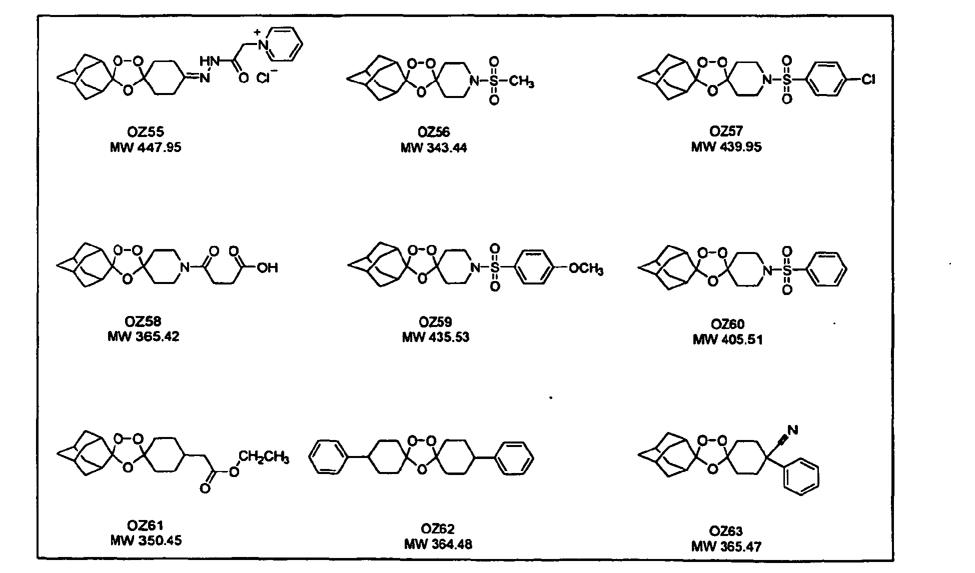

### OZ Series 8 (OZ64-OZ72)

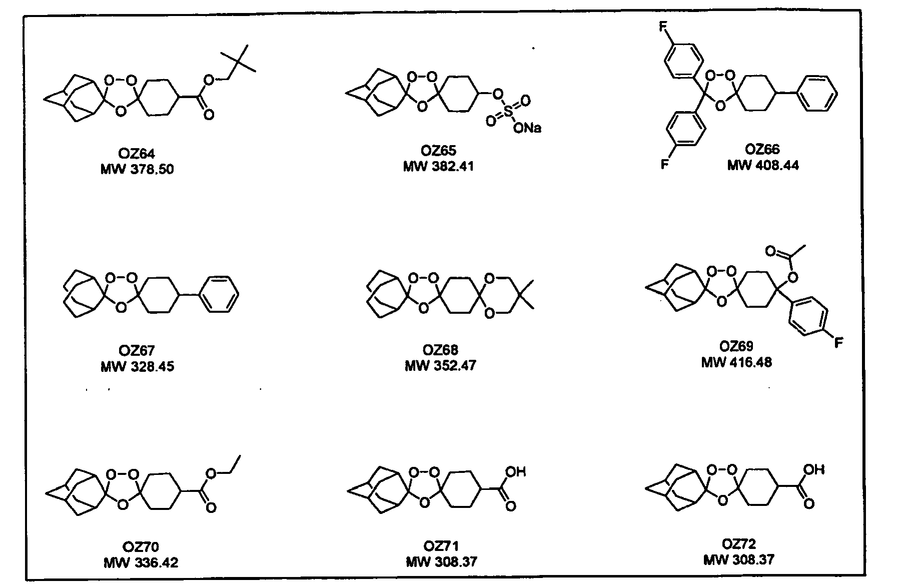

### OZ Series 9 (OZ73-OZ81)

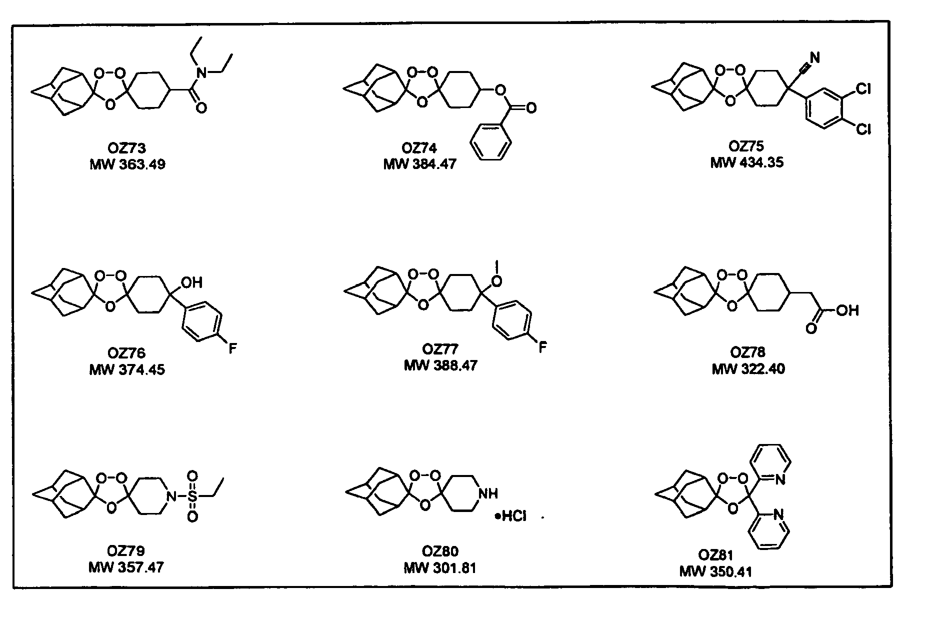

### OZ Series 10 (OZ82-OZ90)

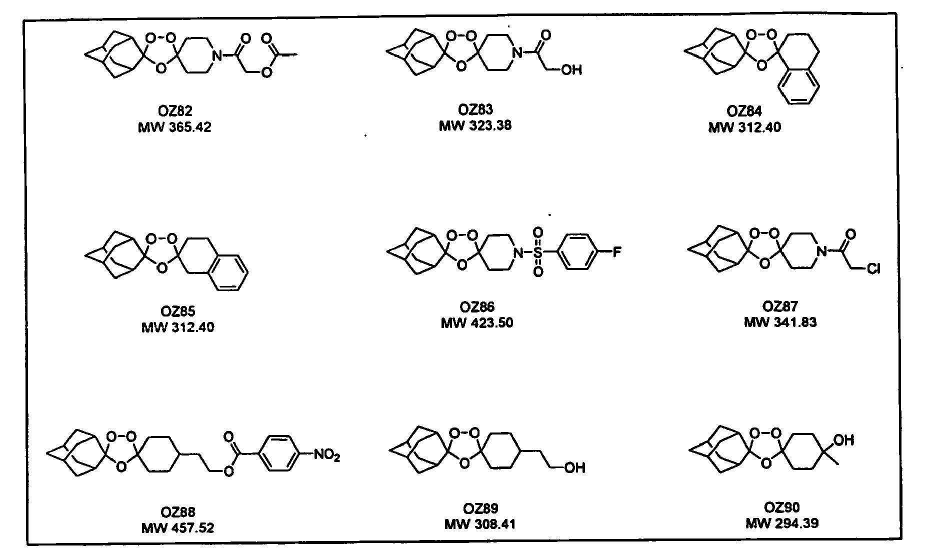

### OZ Series 11 (OZ91-OZ99)

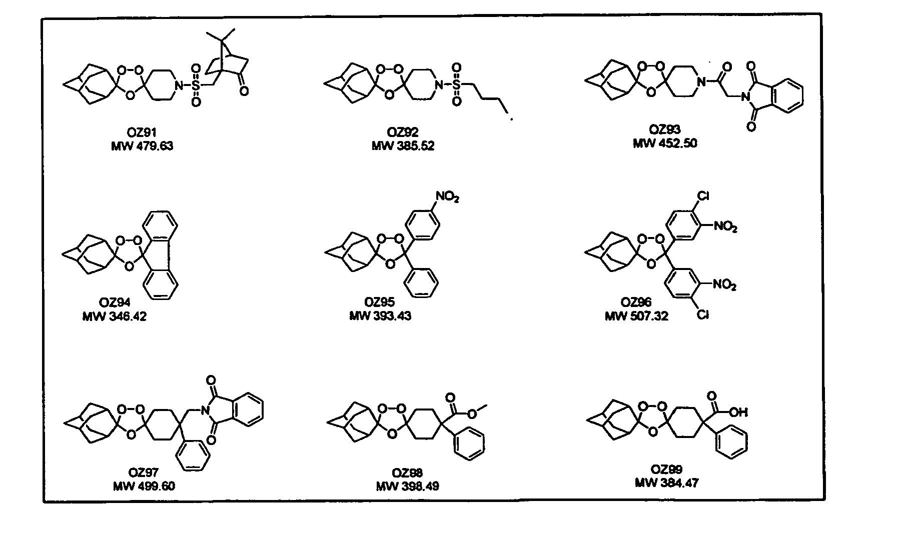

### OZ Series 12 (OZ100-OZ108)

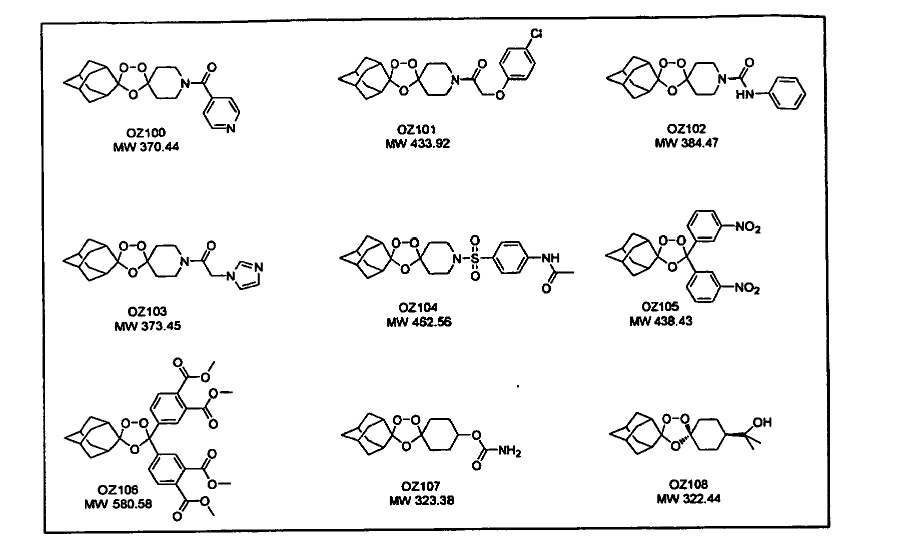

### OZ Series 13 (OZ109-OZ117)

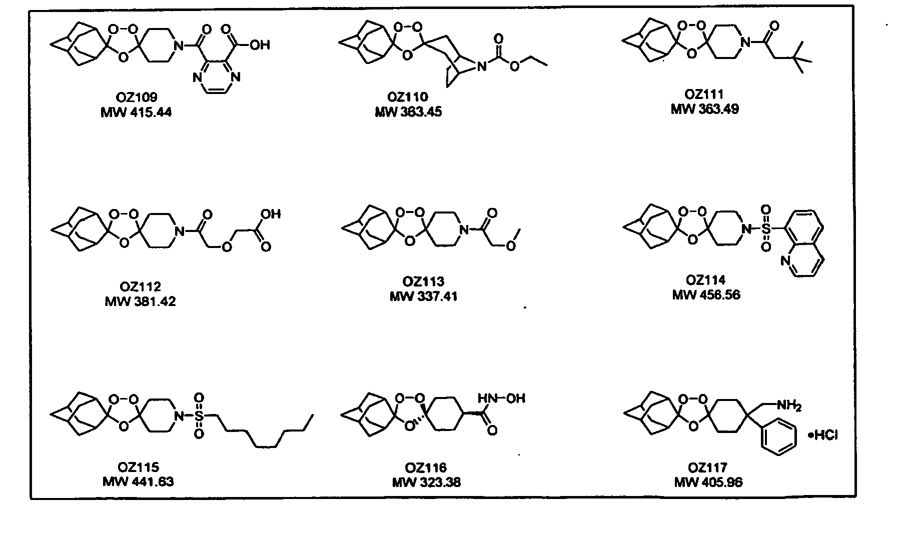

### OZ Series 14 (OZ118-OZ126)

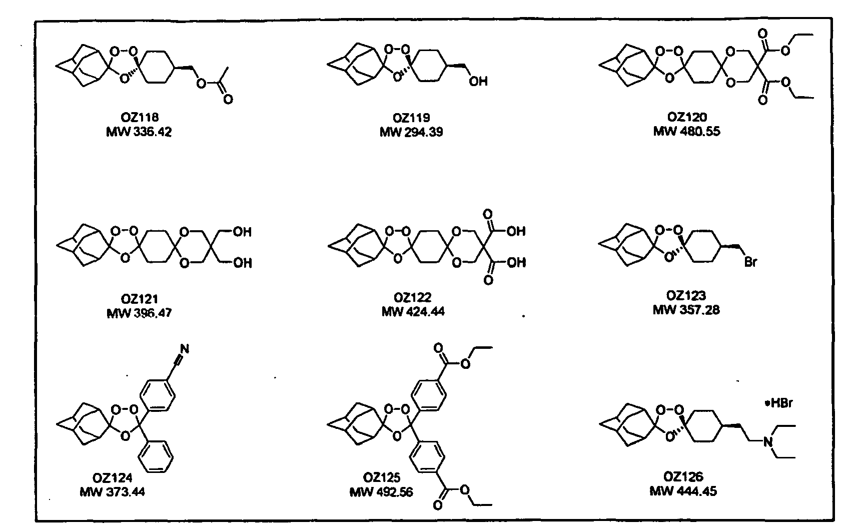

### OZ Series 15 (OZ127-OZ135)

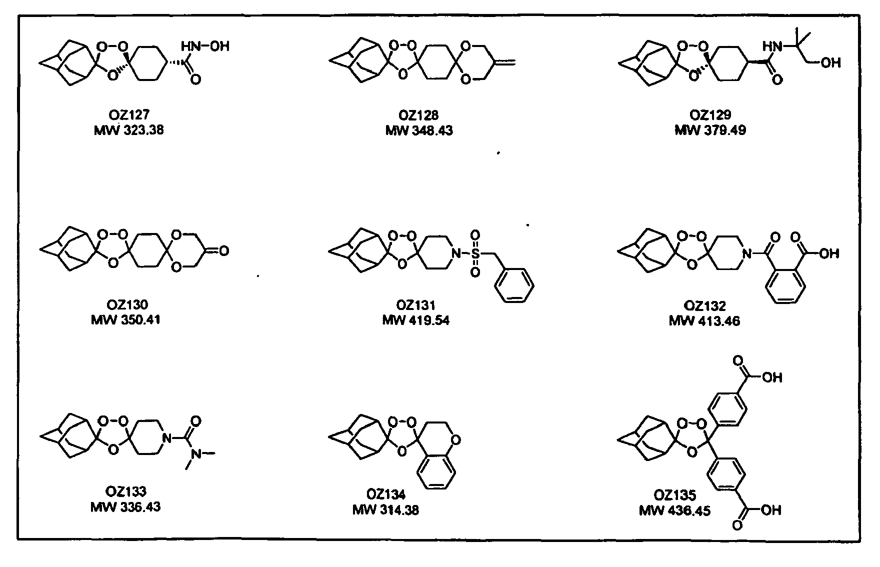

### OZ Series 16 (OZ136-OZ144)

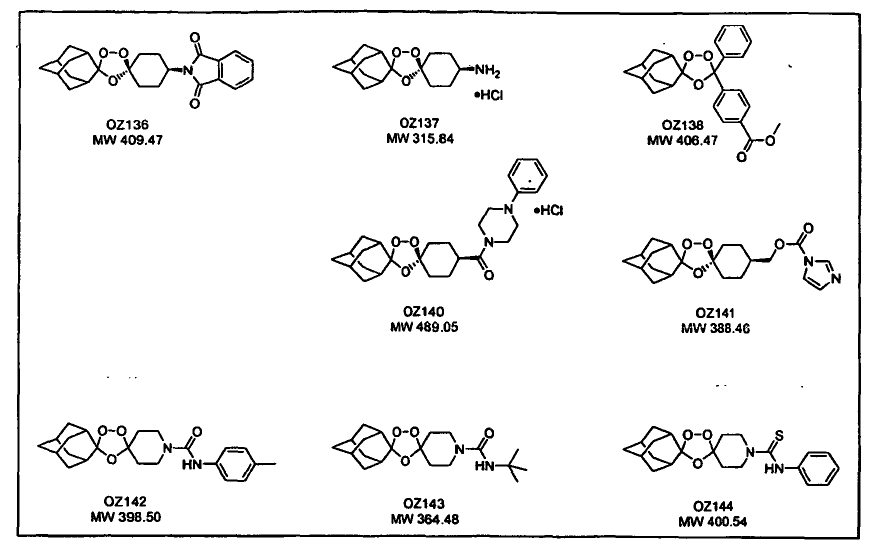

### OZ Series 17 (OZ145-OZ153)

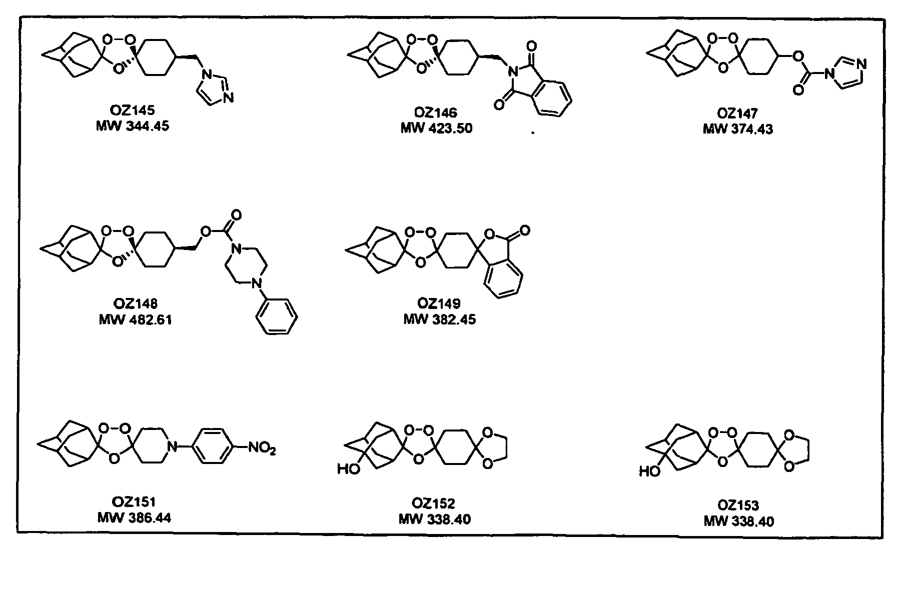

### OZ Series 18 (OZ154-OZ162)

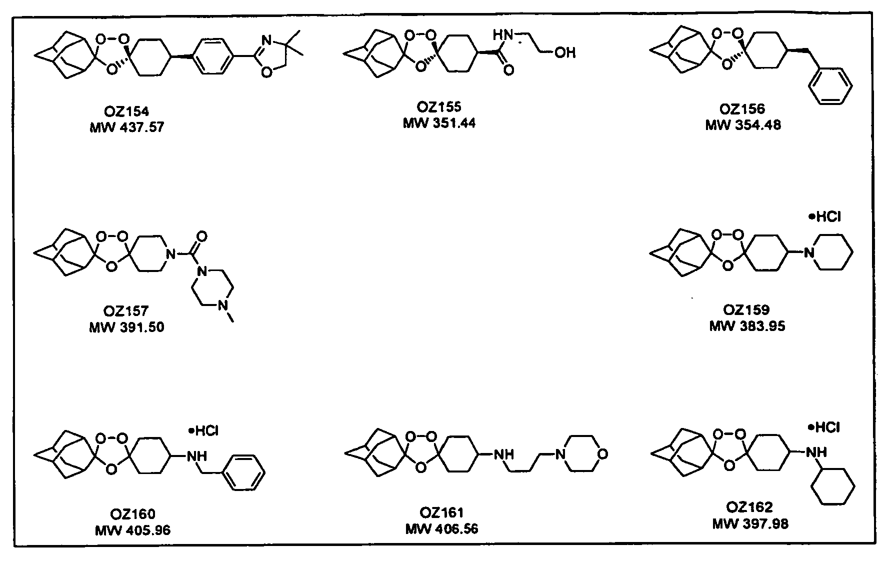

### OZ Series 19 (OZ163-OZ171)

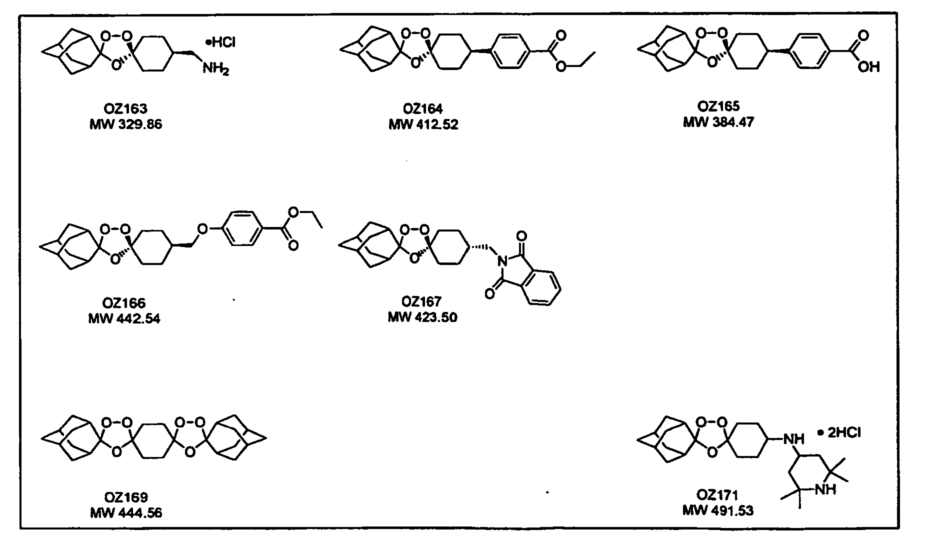

### OZ Series 20 (OZ172-OZ180)

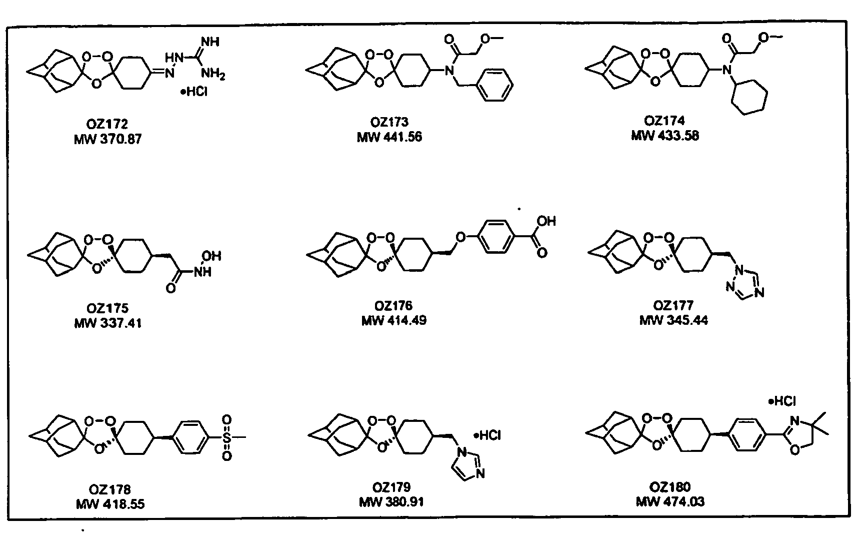

### OZ Series 21 (OZ181-OZ189)

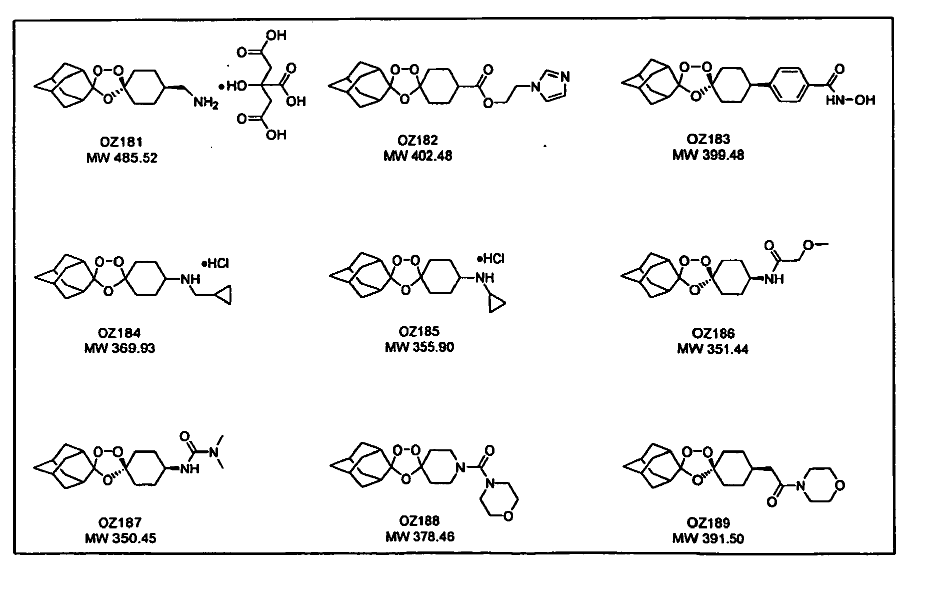

### OZ Series 22 (OZ190-OZ198)

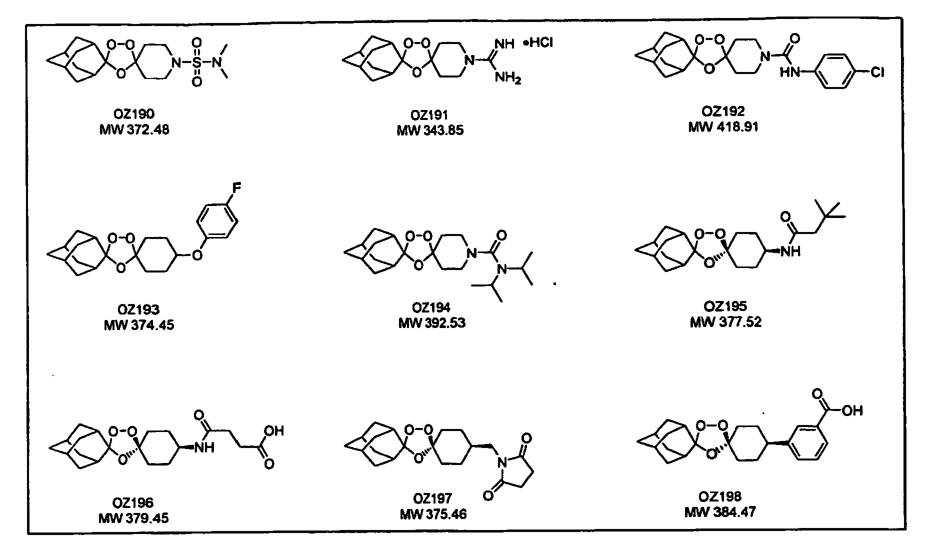

### OZ Series 23 (OZ199-0Z207)

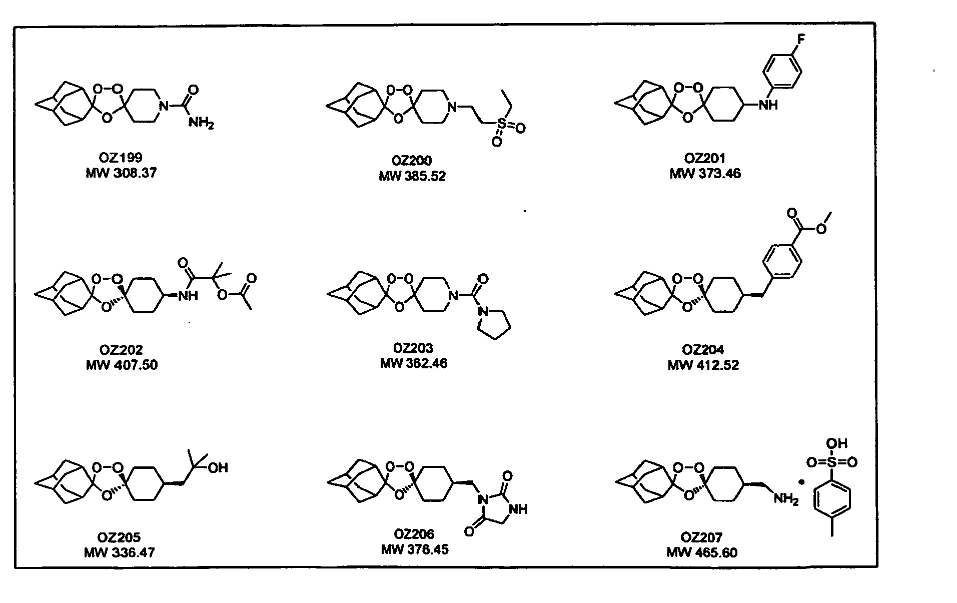

### OZ Series 24 (OZ208-OZ216)

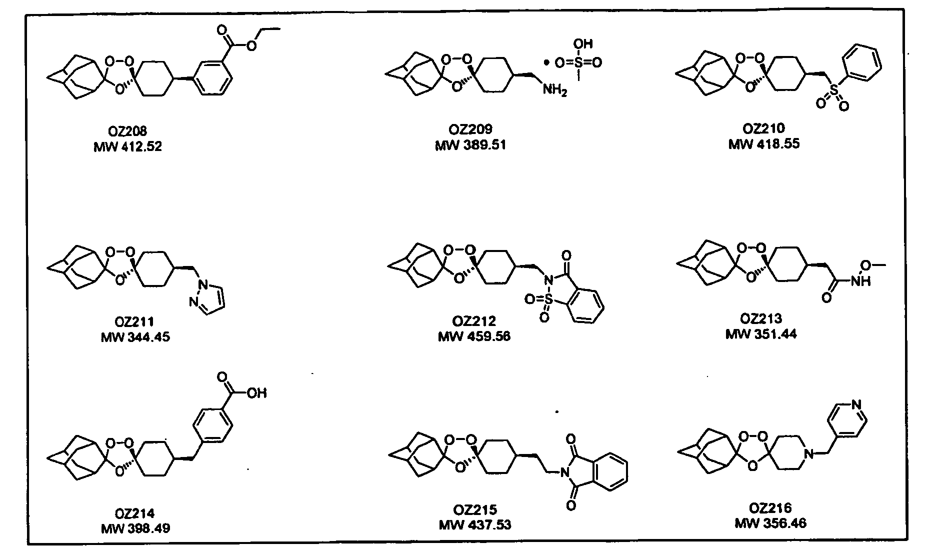

### OZ Series 25 (OZ217-OZ225)

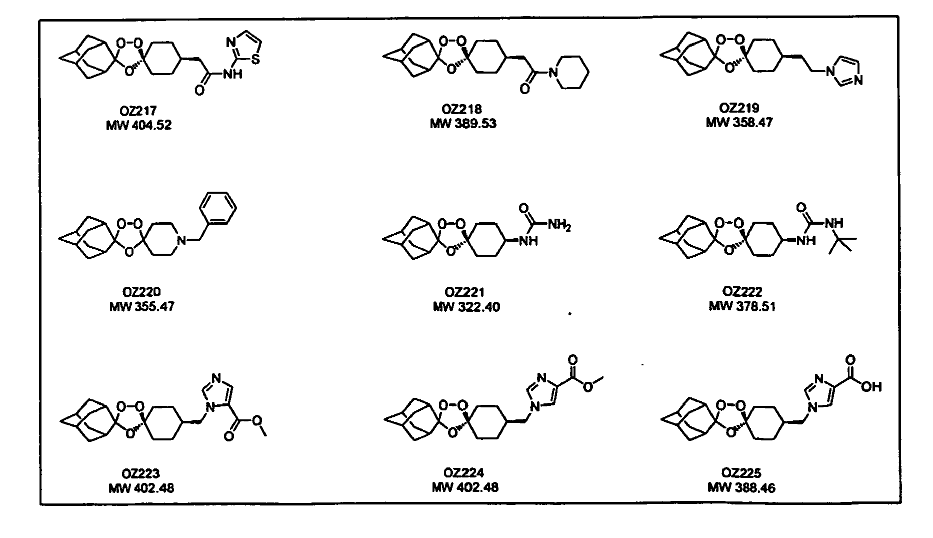

### OZ Series 26 (OZ226-OZ234)

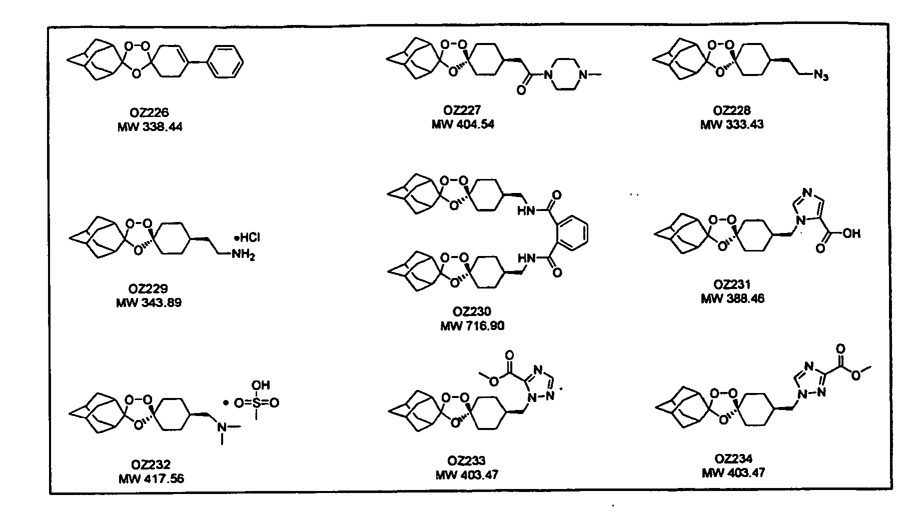

### OZ Series 27 (OZ235-OZ243)

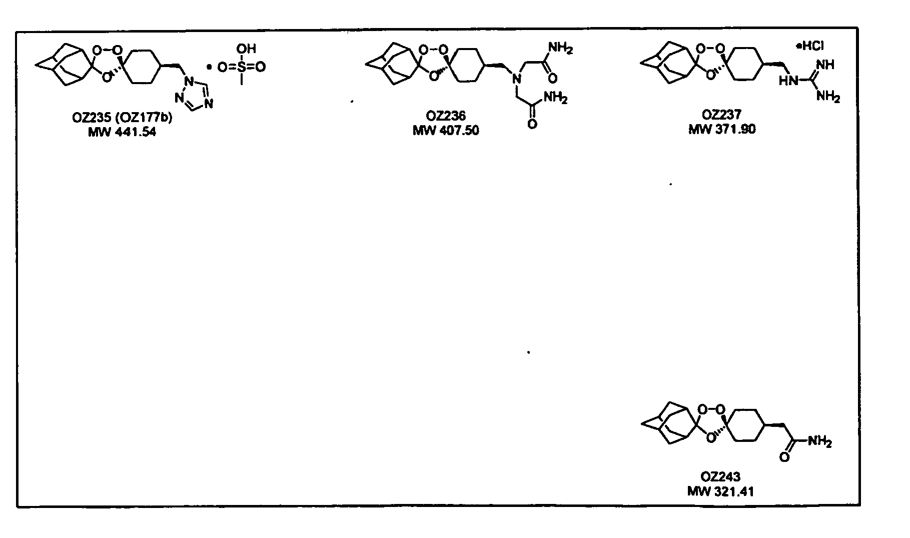

### OZ Series 28 (OZ244-OZ252)

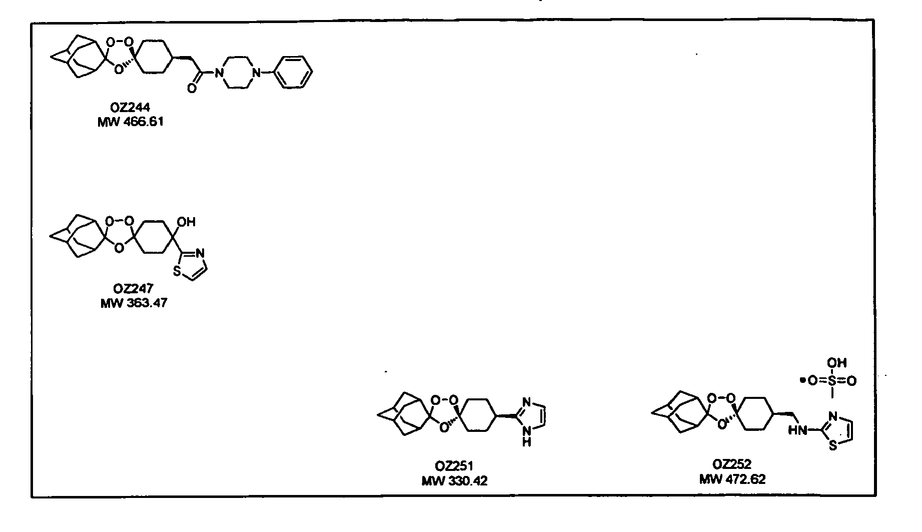

### OZ Series 29 (OZ253-OZ261)

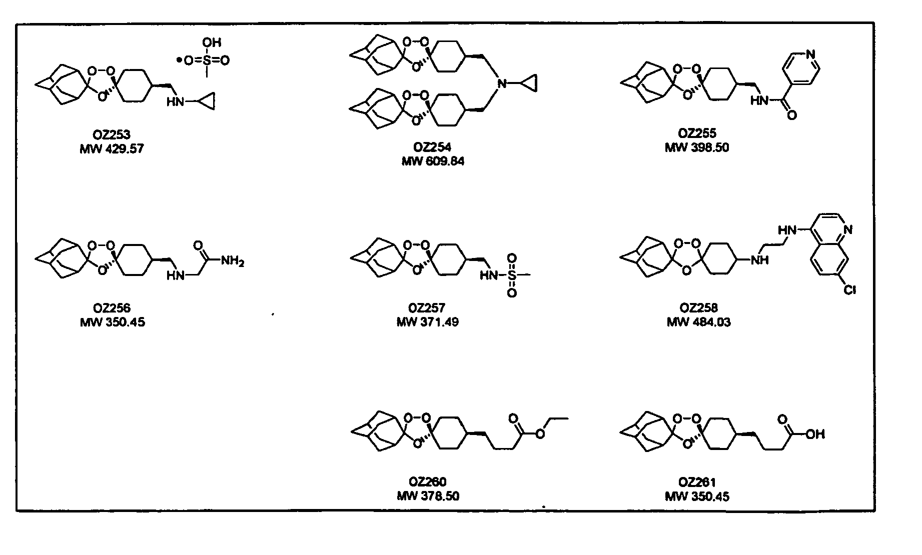

### OZ Series 30 (OZ262-OZ270)

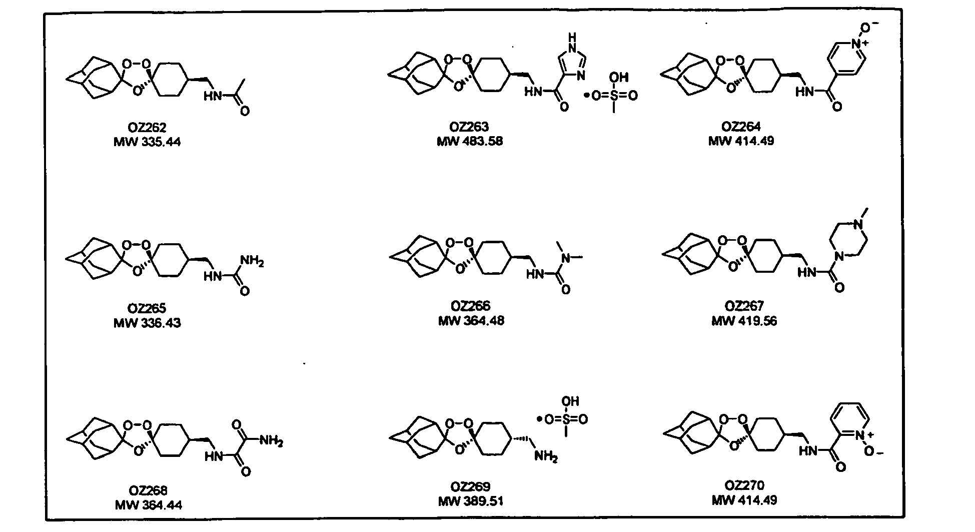

The prototype trioxolanes of this invention are OZ03 and OZ05. Preferred compounds identified thus far include OZ03, OZ05, OZ11, OZ25, OZ27, OZ61, OZ71, OZ78, OZ127, OZ145, OZ156, OZ163, OZ175, OZ177, OZ179, OZ181, OZ189, OZ205, OZ207, OZ209, OZ210, OZ219, OZ227, OZ229, OZ235, OZ255, OZ256, OZ257, OZ263, OZ264, OZ265, OZ266, OZ267, OZ268, OZ269, and OZ270. The most preferred compounds are OZ78, OZ163, OZ181, OZ207, OZ209, OZ255, OZ256, OZ257, OZ263, OZ264, and OZ267. In general, the highest *in vitro* potency against malarial parasites is obtained for trioxolanes functionalized or substituted at the 4-position of the spirocyclohexyl ring. As a general rule, non-symmetrical, achiral trioxolanes are also preferred.

Notable features of these spiro and dispiro 1,2,4-trioxolanes in comparison to the artemisinin semisynthetic derivatives are their structural simplicity and ease of synthesis. For example, dispiro trioxolanes may be easily synthesized by the coozonolysis of the *O-*methyl oximes of cycloalkanones in the presence of the requisite cycloalkanone derivatives according to the method of Griesbaum et al. (1997a; 1997b) as illustrated below for the symmetrical dispiro cyclohexyl trioxolane:

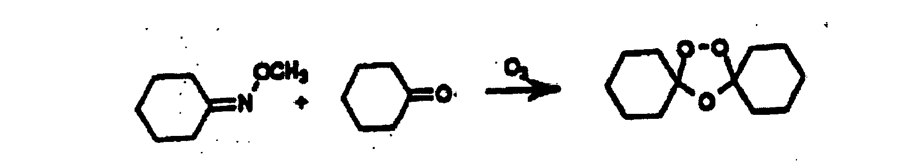

If yields are low in this coozonolysis reaction, yields can improve dramatically when the *O*-methyloxime and ketone are "reversed." This novel procedure provides a uniquely convenient method to synthesize spiro and dispiro trioxolanes. The trioxolanes may be purified by crystallization or by flash column chromatography. Their structures and purity may be confirmed by analytical HPLC, ¹H and ¹³C NMR, IR, melting point and elemental analysis.

Recently, Griesbaum et al. (1997b) discovered that tetrasubstituted 1,2,4-trioxolanes are conveniently obtained by ozonolysis of *O*-alkyl ketone oximes in the presence of carbonyl compounds. Advantages of the oxime ether route over the alkene approach include convenient synthesis of starting materials (oxime ethers vs. tetrasubstituted alkenes), higher yield and selectivity of formation of desired trioxolanes by the judicious selection of paired reaction substrates.

Formation of a trioxolane from an oxime ether and a ketone is presumed to be a three-step process. The sequence begins by the electrophilic addition of ozone to the oxime double bond to form a primary ozonide. Second, the very unstable primary adduct fragments to a reactive carbonyl oxide driven in part by the concomitant expulsion of the relatively stable methyl nitrite. Third, the carbonyl oxide undergoes a [3 + 2] cycloaddition with a ketone to give the secondary ozonide or 1,2,4-trioxolane. It remains to be 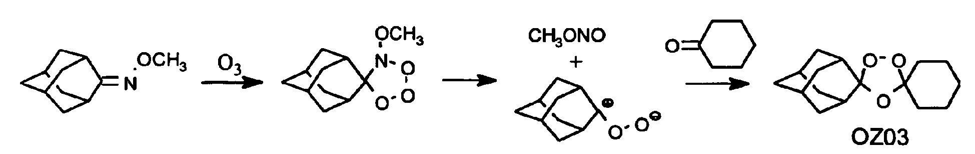 determined whether this is a stepwise or a concerted recombination process.

As illustrated above by the synthesis of OZ03, all of the target dispiro trioxolanes contain a spiroadamantane and can be synthesized by the coozonolysis of adamantanone *O-*methyl oxime in the presence of the requisite cycloalkanone derivative. The preferred reaction solvents for the coozonolysis reactions are hydrocarbon solvents such as pentane or cyclohexane; more polar solvents tend to decrease the yield of the reaction. When ketones are not readily soluble in pentane or cyclohexane, a mixed solvent (pentane/methylene chloride) or methylene chloride alone may be used. Several factors govern the ratio of oxime ether to ketone. In some reactions, in order to avoid diperoxide (1,2,4,5-tetraoxane) formation, to preclude diozonide formation from diketones, and to promote the reaction with readily pentane soluble ketones, excess ketone (2:1) is used. Most commonly in the discovery synthesis stage, and especially in cases where ketones are not readily soluble in pentane, expensive, or difficult to remove in the reaction workup, a 1:1 ratio of ketone to oxime ether may be used. In large scale trioxolane syntheses, a 1.5-fold excess of oxime ether can be used to achieve higher conversions of ketones into the desired product trioxolanes without causing purification problems.

There are several examples of where post-ozonolysis transformations were used to obtain trioxolane target compounds difficult, or in some cases, impossible to obtain directly (Kashima et al., 1987) by the coozonolysis method. Trioxolane tertiary alcohols OZ90 and 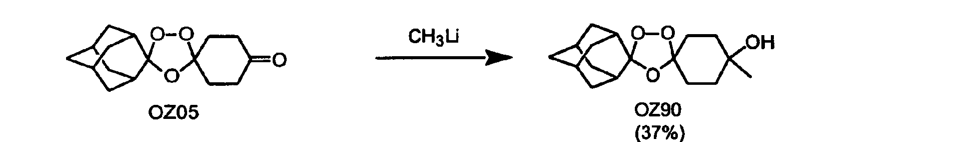 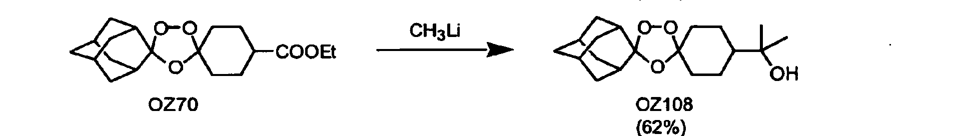 OZ108 can be obtained by methyllithium treatment of trioxolane ketone OZ05 and trioxolane ester OZ70, respectively. In other reactions, trioxolane lactone OZ17 and trioxolane alcohol OZ32 were obtained by treatment of OZ05 with *m*-CPBA and sodium borohydride, respectively. In addition, various oxime ethers, hydrazones, ketals, and amines (reductive amination with sodium triacetoxyborohydride) were also obtained from trioxolane ketone OZ05 in good to excellent yields. In the examples noted above, it is evident that troxolane ketone OZ05 is a key intermediate as its ketone functional group provides a convenient means for functional group transformation.

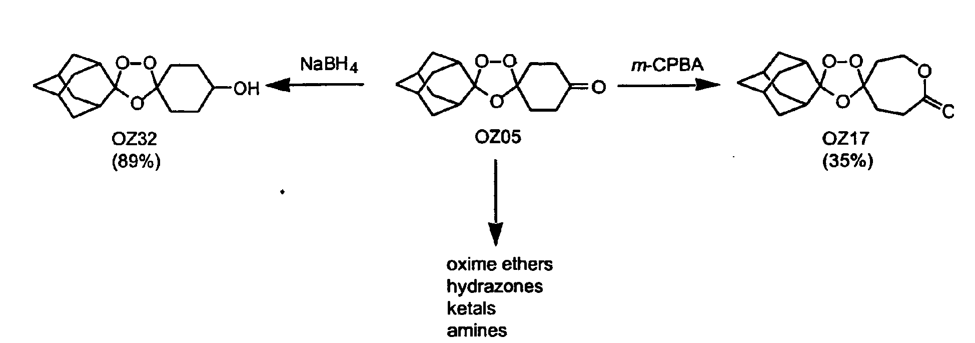

Further evidence of the stability of these trioxolanes to reducing agents is shown by the reduction of trioxolane esters OZ70 and OZ61 into their corresponding trioxolane alcohols OZ119 and OZ89 with a mixture of lithium borohydride and lithium triethylborohydride, and the hydrazinolysis of the trioxolane phthalimides OZ136 and 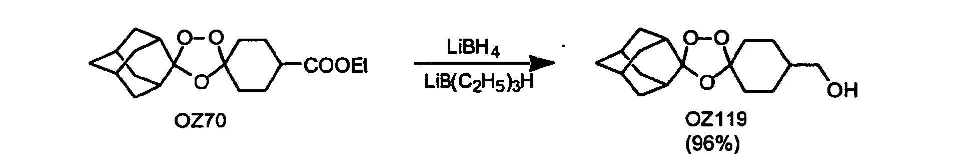 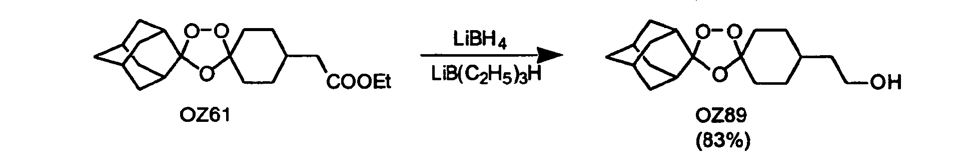 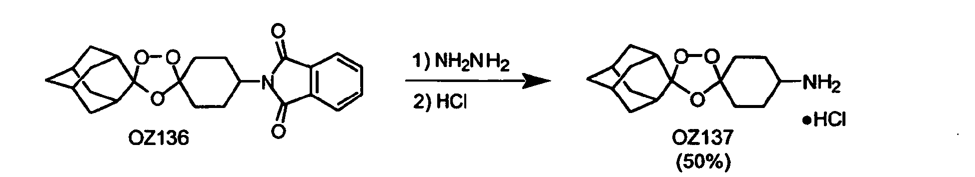 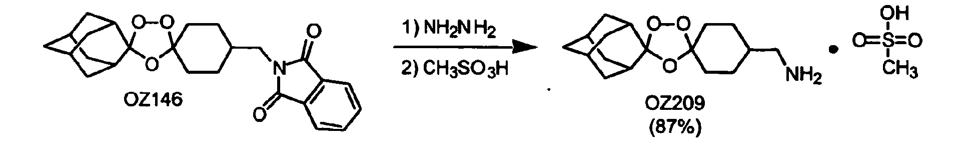 OZ146 into their corresponding trioxolane amines OZ137 and OZ209.

As shown in the examples below, trioxolane esters can be conveniently converted into their corresponding trioxolane acids.

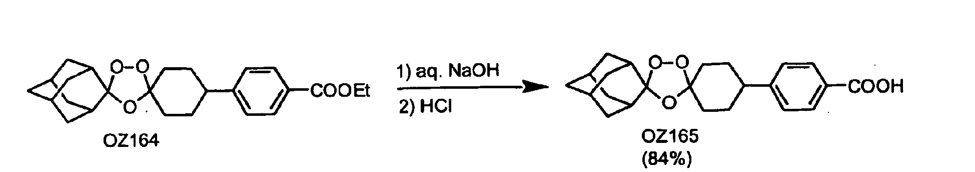

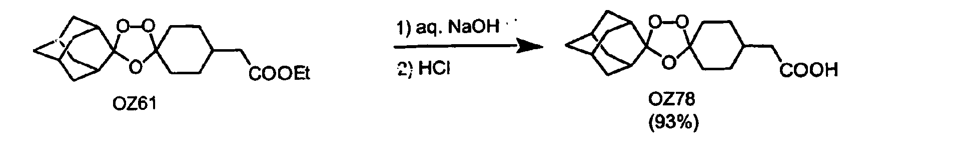

In addition to trioxolane ketone OZ05, trioxolane amine OZ209, trioxolane ester OZ61 and trioxolane acid OZ78, trioxolane alcohols OZ119 and OZ89 and their corresponding mesylates (no assigned OZ #s) have and will continue to be key intermediates for post-ozonolysis synthetic transformations. A recent example is the 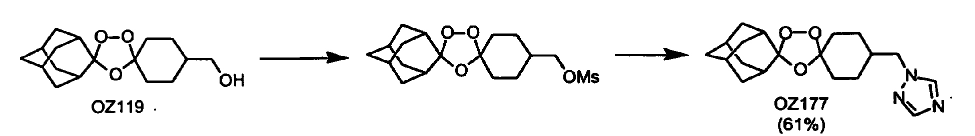 synthesis of trioxolane triazole OZ177 in a reaction between the mesylate derivative of OZ119 and the sodium salt of 1,2,4-triazole.

It has been found that the coozonolysis method using oxime methyl ethers offers a rapid, flexible, and predictable access to structurally diverse trioxolanes. In fact, several key trioxolanes that have served as important building blocks have been prepared in large scale including OZ05 (100 mmol), OZ61 (100 mmol), and OZ146 (60 mmol), with no decrease in reaction yields over the usual 5-10 mmol scale. Furthermore, both OZ61 and OZ146 can be conveniently isolated as white solids by addition of ethanol to the crude reaction mixtures.

Differential scanning calorimetry (DSC) experiments (Cammenga, and Epple, 1995) reveal that these compounds have good thermal stability, comparable to artemisinin. The average Tm, dec was 160 ± 15°C compared to a Tm, dec of 181 °C for artemisinin. It is presumed that thermal decomposition of these trioxolanes was initiated by formation of a 1,5 diradical produced by homolytic cleavage of the peroxide bond of the trioxolane ring.

Since most of the target trioxolanes contain the symmetrical spiroadamantane structural framework, their stereochemistry is largely a function of the starting material ketone structure or reagents used in post-ozonolysis reactions. For OZ27 and other similarly 1,4-substituted trioxolanes, two achiral diastereomers are possible. However, as exemplified by OZ27, the majority of these trioxolanes were isolated as single achiral diastereomers rather than as mixtures of two achiral diastereomers. For example, in OZ27, no chirality is present since the trioxolane ring and phenyl substituent are in a 1,4 relationship in a six membered ring. Such compounds possess a plane of symmetry. 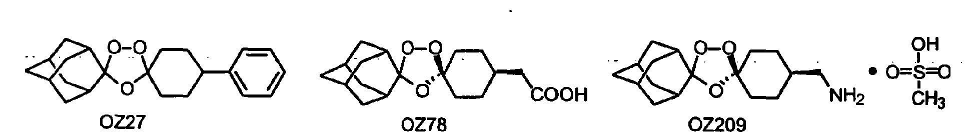 As determined by X-ray crystallography, the assignment of stereochemistry for OZ78, OZ209 and their derivatives was determined to be *cis* where the peroxide oxygens are in an axial position.

The following cycloalkanone and cycloalkanedione starting materials can be obtained from Aldrich Chemical Co. or from TCI American Organic Chemicals: cyclohexanone, cyclododecanone, 1,4-cyclohexanedione, 2-adamantanone, bicyclo[3.3.1]nonan-9-one, tetrahydro-4*H*-pyran-4-one, 1-carboethoxy-4-piperidone, 1-benzoyl-4-piperidone, α-tetralone, β-tetralone, bicyclo[3.3.1]nonan-3,7-dione, 1,4-cyclohexanedione-mono-2,2-dimethyltrimethylene ketal, cis-bicyclo[3.3.0]octane-3,7-dione, and 4-carboethoxycyclohexanone.

The cycloalkanone starting materials may also be synthesized. For instance, the inventors have synthesized 4,4-dimethylcyclohexanone and 4,4-diphenylcyclohexanone by catalytic hydrogenation (Augustine, 1958) of the commercially available enones. Also, 2-carboethoxyethylcyclohexanone was synthesized by treatment of the pyrrolidine enamine of cyclohexanone with ethyl acrylate (Stork et al., 1963). Persons skilled in the art can readily ascertain other appropriate means of synthesizing the starting materials and compounds in accordance with this invention.

The spiro and dispiro trioxolane compositions of the present invention may be generally used for the prophylaxis and treatment of malaria. The trioxolane compositions of the present invention are administered along with a pharmaceutically acceptable carrier. Any pharmaceutically acceptable carrier may be generally used for this purpose, provided that the carrier does not significantly interfere with the stability or bioavailability of the trioxolane compounds of this invention.

The trioxolanes of this invention can be administered in any effectively pharmaceutically acceptable form to warm blooded animals, including human and other animal subjects, e.g. in topical, lavage, oral, suppository, parenteral, or infusible dosage forms, as a topical, buccal, sublingual, or nasal spray or in any other manner effective to deliver the agents. The route of administration will preferably be designed to optimize delivery and/or localization of the agents to target cells.

In addition to the active compounds i.e. the trioxolanes, the pharmaceutical compositions of this invention may contain suitable excipients and auxiliaries which facilitate processing of the active compounds into preparations which can be used pharmaceutically. Oral dosage forms encompass tablets, capsules, and granules. Preparations which can be administered rectally include suppositories. Other dosage forms include suitable solutions for administration parenterally or orally, and compositions which can be administered buccally or sublingually.

The pharmaceutical preparations of the present invention are manufactured in a manner which is itself well known in the art. For example the pharmaceutical preparations may be made by means of conventional mixing, granulating, dragee-making, dissolving, lyophilizing processes. The processes to be used will depend ultimately on the physical properties of the active ingredient used.

Suitable excipients are, in particular, fillers such as sugars for example, lactose or sucrose mannitol or sorbitol, cellulose preparations and/or calcium phosphates, for example, tricalcium phosphate or calcium hydrogen phosphate, as well as binders such as starch, paste, using, for example, maize starch, wheat starch, rice starch, potato starch, gelatin, gum tragacanth, methyl cellulose, hydroxypropylmethylcellulose, sodium carboxymethylcellulose, and/or polyvinyl pyrrolidone. If desired, disintegrating agents may be added, such as the above-mentioned starches as well as carboxymethyl starch, cross-linked polyvinyl pyrrolidone, agar, or alginic acid or a salt thereof, such as sodium alginate. Auxiliaries are flow-regulating agents and lubricants, for example, such as silica, talc, stearic acid or salts thereof, such as magnesium stearate or calcium stearate and/or polyethylene glycol. Oral dosage forms may be provided with suitable coatings which, if desired, may be resistant to gastric juices.

For this purpose concentrated sugar solutions may be used, which may optionally contain gum arabic, talc, polyvinylpyrrolidone, polyethylene glycol and/or titanium dioxide, lacquer solutions and suitable organic solvents or solvent mixtures. In order to produce coatings resistant to gastric juices, solutions of suitable cellulose preparations such as acetylcellulose phthalate or hydroxypropylmethylcellulose phthalate, dyestuffs and pigments may be added to the tablet coatings, for example, for identification or in order to characterize different combination of compound doses.

Other pharmaceutical preparations which can be used orally include push-fit capsules made of gelatin, as well as soft, sealed capsules made of gelatin and a plasticizer such as glycerol or sorbitol. The push-fit capsules can contain the active compounds in the form of granules which may be mixed with fillers such as lactose, binders such as starches, and/or lubricants such as talc or magnesium stearate and, optionally, stabilizers. In soft capsules, the active compounds are preferably dissolved or suspended in suitable liquids, such as fatty oils, liquid paraffin, or liquid polyethylene glycols. In addition stabilizers may be added. Possible pharmaceutical preparations which can be used rectally include, for example, suppositories, which consist of a combination of the active compounds with the suppository base. Suitable suppository bases are, for example, natural or synthetic triglycerides, paraffin hydrocarbons, polyethylene glycols, or higher alkanols. In addition, it is also possible to use gelatin rectal capsules which consist of a combination of the active compounds with a base. Possible base material include for example liquid triglycerides, polyethylene glycols, or paraffin hydrocarbons.

Suitable formulations for parenteral administration include aqueous solutions of active compounds in water-soluble or water-dispersible form. In addition, suspensions of the active compounds as appropriate oily injection suspensions may be administered. Suitable lipophilic solvents or vehicles include fatty oils for example, sesame oil, or synthetic fatty acid esters, for example, ethyl oleate or triglycerides. Aqueous injection suspensions may contain substances which increase the viscosity of the suspension, including for example, sodium carboxymethyl cellulose, sorbitol and/or dextran. Such compositions may also comprise adjuvants such as preserving, wetting, emulsifying, and dispensing agents. They may also be sterilized, for example, by filtration through a bacteria-retaining filter, or by incorporating sterilizing agents into the compositions. They can also be manufactured in the form of sterile solid compositions which can be dissolved or suspended in sterile water, saline, or other injectable medium prior to administration.

In addition to administration with conventional carriers, active ingredients may be administered by a variety of specialized delivery drug techniques which are known to those of skill in the art, such as portable infusion pumps.

The trioxolane compositions of the present invention are administered along with a pharmaceutically acceptable carrier in an amount sufficient to prevent malarial infection and/or treat an active infection. The trioxolane compounds of this invention have extremely low toxicity and a low degree of side effects even at high doses. The dosing range of the trioxolane compositions will vary depending on a number of factors, such as whether it is used for prophylaxis or treatment of an active infection, route of administration, dosing schedule, etc. In general, the therapeutic dose of trioxolane may range between about 0.1-1000 mg/kg/day, with between about 1-100 mg/kg/day being preferred. The foregoing doses may be administered as a single dose or may be divided into multiple doses for administration. The trioxolane compositions may be administered once to several times daily. For malaria prevention, a typical dosing schedule could be, for example, 2.0-1000 mg/kg weekly beginning 1-2 weeks prior to malaria exposure taken up until 1-2 weeks post-exposure.

The spiro and dispiro trioxolanes of this invention have been found to be effective in the treatment of schistosomiasis. Schistosomiasis ranks second behind malaria in terms of socioeconomic and public health importance in tropical and subtropical areas. The disease is endemic in 74 developing countries, infecting more than 200 million people in rural agricultural and peri-urban areas. An estimated 500-600 million people worldwide are at risk from the disease.

The major forms of human schistosomiasis are caused by five species of water-borne flatworm, or blood flukes, called schistosomes. One of these species is *Schistosoma mansoni,* which has been reported in 53 countries in Africa, the Eastern Mediterranean, the Caribbean, and South America. The parasites enter the body through contact with infested surface water, primarily among people engaged in agriculture and fishing. The parasites normally infect the host during the cercaria, or larval stage. Once inside the host, the cercaria develop into adults or schistosomes.

Current treatments for schistosomiasis have focused primarily on prophylaxis, i.e. prevention of host infection by cercaria. Currently, praziquantel is the most widely used drug for treatment of schistosomiasis. While artemether has demonstrated activity in the prophylaxis of schistosomiasis, it has not shown any activity against adult *S. mansoni.*

It has now been unexpectedly discovered that the spiro and dispiro trioxolanes of this invention are active against both cercaria and adult *S. mansoni, S. japonicum* when administered in the dosages and manner outlined above with respect to treatment of malarial parasites. It is also believed the trioxolanes of this invention will be active against *S. haematobium.* Preferred compounds identified for use in the treatment of schistosomiasis include OZ03, OZ05, OZ11, OZ16, OZ23, OZ25, OZ27, OZ32, OZ71, OZ78, OZ89, OZ90, OZ119, OZ145, OZ163, OZ205, OZ207, OZ209, OZ210, OZ219, OZ227, OZ229, OZ235, OZ255, OZ256, OZ257, OZ263, OZ264, OZ265, OZ266, OZ267, OZ268, OZ269, and OZ270. Most preferred compounds are OZ05, OZ23, OZ25, OZ71, OZ78, OZ89, OZ119, OZ163, OZ205, OZ207, and OZ209. Preferred dosing levels of the spiro and dispiro trioxolanes are about 100-200 mg/kg/day orally. The prototype trioxolanes of this invention are OZ03 and OZ05.

The spiro and dispiro trioxolanes of this invention may also have effectiveness in the treatment of cancer. Compounds having an endoperoxide moiety that is reactive with heme and iron have shown an ability to kill cancer cells. (See e.g. U.S. Pat. No. 5,578,637, the disclosure of which is hereby incorporated by reference). As noted with respect to artemisinin, trioxolanes' mechanism of action against malarial parasites is based on the ability of trioxolane compounds to react with the iron in free heme molecules in malaria parasites, with the generation of free radicals leading to cellular destruction. Similarly, trioxolanes are selective against cancer cells due to the higher concentration of transferrin receptors on cancer cell membranes that pick up iron at a higher rate than normal cells. In the presence of the trioxolanes of this invention, the cancer cells will accumulate high concentrations of free radicals, leading to cell death. For cancer treatment, the trioxolanes of this invention may be administered in the doses and manner outlined above.

Other drugs besides trioxolanes which are compatible with the carrier ingredients may also be incorporated into the carrier. Such drugs may be readily ascertained by those of ordinary skill in the art and may include, for instance, antibiotics, other antimalarials, antiinflammatory agents, etc.

It is understood that the present invention contemplates the use of not only the above-stated trioxolane compounds themselves, but their prodrugs which metabolize to the compound and the analogues and biologically active salt forms thereof, as well as optical isomers which provide the same pharmaceutical results.

The following examples are offered to illustrate but not limit the invention. Thus, they are presented with the understanding that various formulation modifications as well as method of delivery modifications may be made and still be within the spirit of the invention.

### EXAMPLE 1

### General Procedure for the Preparation of 1,2,4-Trioxolanes

**Synthesis of *O*-methyl 2-adamantanone oxime (representative procedure).** To a solution of 2-adamantanone (4.51 g, 30 mmol) in methanol (30 ml) were added pyridine (4.5 ml) and methoxylamine hydrochloride (3.76 g, 45.0 mmol). The reaction mixture was stirred at room temperature for 48 h, concentrated in vacuo, and diluted with CH₂Cl₂ (50 ml) and water (50 ml). The organic layer was separated, and the aqueous layer extracted with CH₂Cl₂ (30 ml). The combined organic extracts were washed with 1 M HCl (30 ml x 2) and saturated aqueous NaCl (30 ml), and dried over MgSO₄. Evaporation in vacuo afforded *O*-methyl 2-adamantanone oxime (4.77 g, 89%) as a colorless solid. mp 70-71 °C; ¹H NMR (300 MHz, CDCl₃) δ 1.60-2.10 (m, 12H), 2.54 (s, 1H), 3.47 (s, 1H), 3.82 (s, 3H).

Ref: Corey, E. J.; Niimura, K.; Konishi, Y.; Hashimoto, S.; Hamada, Y. A New Synthetic Route to Prostaglandins. *Tetrahedron Lett.* **1986,** *27,* 2199-2202.

***O*-Methyl cyclohexanone oxime.** Yield, 76%; colorless oil; ¹H NMR (300 MHz, CDCl₃) δ 1.40-1.80 (m, 6H), 2.20 (t, J = 6.0 Hz, 2H), 2.45 (t, J = 6.1 Hz, 2H), 3.81 (s, 3H).

***O*-Methyl cyclododecanone oxime.** Yield, 98%; colorless oil; ¹H NMR (500 MHz, CDCl₃) δ 1.20-1.49 (m, 14H), 1.50-1.60 (m, 2H), 1.61-1.70 (m, 2H), 2.22 (t, J = 6.8 Hz, 2H), 2.35 (t, J = 6.6 Hz, 2H), 3.81 (s, 3H).

***O*-Methyl 3,3,5,5-tetramethylcyclohexanone oxime.** Yield, 91%; colorless oil; ¹H NMR (500 MHz, CDCl₃) δ 0.96 (s, 6H), 0.97 (s, 6H), 1.33 (s, 2H), 1.95 (s, 2H), 2.20 (s, 2H), 3.80 (s, 3H).

***O*-Methyl 4-phenylcyclohexanone oxime.** Yield, 92%; colorless solid; mp 45-47°C; ¹H NMR (500 MHz, CDCl₃) δ 1.57-1.76 (m, 2H), 1.82-1.92 (m, 1H), 1.99-2.13 (m, 2H), 2.19-2.30 (m, 1H), 2.47-2.56 (m, 1H), 2.72-2.81 (m, 1H), 3.32-3.42 (m, 1H), 3.85 (s, 3H), 7.17-7.34 (m, 5H).

***O*-Methyl bicyclo[3.3.1]nonan-9-one oxime.** Yield, 96%; colorless oil; ¹H NMR (500 MHz, CDCl₃) δ 1.46-1.62 (m, 2H), 1.72-2.11 (m, 10H), 2.47 (br s, 1H), 3.40 (br s, 1H), 3.82 (s, 3H).

**1-(*p*-Toluenesulfonyl)-4-piperidone.** To a solution of 4-piperidone monohydrate hydrochloride (7.68 g, 50 mmol) in methylene chloride (50 ml) were added sequentially *p-*toluenesulfonyl chloride (10.50 g, 55.07 mmol) and triethylamine (21 ml). The mixture was stirred at room temperature for 16 h before being quenched with water (100 ml). The organic layer was washed with 1 M HCl (100 ml) and brine (100 ml), and dried over sodium sulfate. Evaporation of the solvent gave the desired ketone (8.60 g, 68%) as a colorless solid. mp 130-132 °C; ¹H NMR (500 MHz, CDCl₃) δ 2.40 (s, 3H), 2.58 (t, J = 6.4 Hz, 4H), 3.38 (t, J = 6.4 Hz, 4H), 7.35 (d, J = 7.8 Hz, 2H), 7.68 (d, J = 8.3 Hz, 2H).

**1-[3-(Ethoxycarbonyl)propionyl]-4-piperidone.** To a solution of 4-piperidone monohydrate hydrochloride (7.68 g, 50 mmol) and triethylamine (21 ml) in methylene chloride (100 ml) was added 3-(ethoxycarbonyl)propionyl chloride (9.87 g, 60 mmol) at 0 °C over a period of 10 min. The mixture was stirred at room temperature for 16 h before being quenched with water (100 ml). The organic layer was separated and the aqueous layer was extracted with methylene chloride (100 ml). The combined organic layers were washed with 1M HCl (100 ml), saturated aqueous sodium bicarbonate (100 ml), and brine (100 ml), dried over sodium sulfate, and concentrated. Purification by flash chromatography (silica gel, 30% ether in hexanes) gave the desired ketone (3.80 g, 33%) as a light yellow oil. ¹H NMR (500 MHz, CDCl₃) δ 1.27 (t, J = 7.3 Hz, 3H), 2.48 (t, J = 6.4Hz, 2H), 2.53 (t, J = 6.4 Hz, 2H), 2.68 (s, 4H), 3.82 (t, J = 6.3 Hz, 2H), 3.82 (t, J = 6.3 Hz, 2H), 4.16 (q, J = 7.3 Hz, 2H).

**1,1-Dioxotetrahydrothiopyran-4-one.** To a solution of tetrahydrothiopyran-4-one (400 mg, 3.45 mmol) in acetonitrile (5 ml) was added aqueous Na₂EDTA (3 ml, 0.0004 M). A mixture of oxone (6.30 g, 10.30 mmol) and sodium bicarbonate (2.70, 32 mmol) was added in small portions to the above solution over a period of 20 min. The slurry was stirred for another 1 h before being quenched with methylene chloride. The organic solvent was decanted and the solid residue was triturated with ethyl acetate (3 x 25 ml). The combined organic layers were dried over sodium sulfate and concentrated to give the desired ketone (0.37 g, 73%) as a colorless solid. mp 170-172 °C (lit. 168-170 °C); ¹H NMR (500 MHz, CDCl₃) 2.99 (t, J = 6.8 Hz, 4H), 3.39 (t, J = 6.8 Hz, 4H). Ref: Yang, D.; Yip, Y.-C.; Jiao, G.-S.; Wong, M.-K. Design of Efficient Ketone Catalysts for Epoxidation by Using the Field Effect. *J. Org. Chem,* 1998, *63*, 895.2-8956.

**Synthesis of 1-benzenesulfonyl-4-piperidone (representative procedure).** To a solution of 4-piperidone monohydrate hydrochloride (4.59 g, 30 mmol), triethylamine (12.5 ml, 90 mmol) in CH₂Cl₂ (50 ml) was added benzenesulfonyl chloride (5.30 g, 30 mmol). The mixture was stirred at 25 °C for 16 h. After evaporation of solvents, the residue was triturated with water (100 ml), filtered, and further purified by recrystallization from hexanes/CH₂Cl₂ (3:1) to afford the desired ketone (5.97 g, 83%) as a colorless solid. mp 116-118°C; ¹H NMR (500 MHz, CDCl₃) δ 2.54 (t, J = 6.4Hz, 4H), 3.41(t, J = 6.4Hz, 4H), 7.58 (d, J = 7.8 Hz, 2H), 7.63 (t, J = 7.0 Hz, 1H), 7.81 (d, J = 7.8 Hz, 2H).

**1-(4-Methoxybenzenesulfonyl)4-piperidone.** Yield, 77%; colorless solid; mp 130-132 °C; ¹H NMR (500 MHz, CDCl₃) δ 2.56 (t, J = 6.4 Hz, 4H), 3.38 (t, J = 6.3 Hz, 4H), 3.95 (s, 3H), 7.00 (d, J = 8.2 Hz, 2H), 7.75 (d, J = 8.2 Hz, 2H).

**1-(4-Chlorobenzenesulfonyl)-4-piperidone.** Yield, 73%; colorless solid; mp 166-168 °C; ¹H NMR (500 MHz, CDCl₃) δ 2.55 (t, J = 6.4 Hz, 4H), 3.41 (t, J = 6.4 Hz, 4H), 7.54 (d, J = 8.3 Hz, 2H), 7.81 (d, J = 8.4 Hz, 2H).

**1-Methanesulfonyl-4-piperidone.** To a suspension of 4-piperidone monohydrate hydrochloride (2.0 g, 13 mmol) and K₂CO₃ (9.0 g, 65.2 mmol) in acetone (40 ml) was added methanesulfonyl chloride (5.96 g, 52.1 mmol) at 0-5 °C. The mixture was stirred at 25 °C for 24 h. The solid material was removed by filtration, and the filtrate was concentrated to dryness. The residue was purified by flash chromatography (silica gel, 80% ether in hexanes) to afford the desired ketone (1.20 g, 52%) as a colorless solid. mp 102-104 °C; ¹H NMR (500 MHz, CDCl₃) δ2.58 (t, J = 6:4 Hz, 4H), 2.90 (s, 3H), 3.60 (t, J = 6.4 Hz, 4H).

**Ethoxycarbonylmethylene triphenylphosphorane.** To a solution of triphenylphosphine (26.20 g, 100 mmol) in benzene (150 ml) was added ethyl bromoacetate (16.70 g, 100 mmol) at 0-5 °C. The mixture was kept at room temperature for 16 h. The resulting phosphonium salt was filtered, washed with benzene (100 ml), and dried. To a solution of the solid in water (200 ml) was added benzene (200 ml), followed by 10% NaOH solution (100 ml). The organic layer was separated, and the aqueous layer was extracted with benzene (200 ml). The combined organic layers were washed with water (100 ml) and brine (100 ml), concentrated to 50 ml in vacuo, and poured onto hexane (200 ml). The precipitate was filtered and dried to afford the desired phosphorane (28.00 g, 80%) as a colorless solid. mp 128-130°C.

**4-Oxocyclohexylideneacetic acid ethyl ester.** To a solution of 1,4-cyclohexanedione (5.00 g, 44.64 mmol) in benzene (100 ml) was added the ylide (15.55 g, 44.68 mole). The mixture was heated under reflux for 12 h. After removal of the solvent by evaporation, the residue was purified by flash chromatography (silica gel, 5% ethyl acetate in hexanes) to afford the ketone ester (5.80 g, 71%) as a colorless oil. ¹H NMR (500 MHz, CDCl₃) δ 1.26 (t, J = 6.4 Hz, 3H), 2.42-2.50 (m, 4H,), 2.60-2.66 (m, 2H), 3.12-3.20 (m, 2H), 4.16 (q, J = 6.4 Hz, 2H), 5.86 (s, 1H).

**4-Oxocyclohexaneacetic acid ethyl ester.** To a solution of the unsaturated ester (2.50g, 13.74 mmol) in ethanol (25 ml) was added Raney nickel (1.0 g). The mixture was stirred at room temperature under H₂ (balloon) for 24 h. After the catalyst was removed by filtration, the filtrate was concentrated to give the alcohol ester, which was used for the Jones' oxidation without further purification. To a solution of the above residue in acetone (20 ml) at 0 °C was added Jones' reagent (6 ml), prepared by dissolving CrO₃ (27.20 g) in concentrated sulfuric acid (25 ml) and further diluting the solution to 100 ml with water. The reaction was stirred at 0 °C for 2 h before being quenched with isopropanol (3 ml). The organic solvent was removed in vacuo and the residue was diluted with ether (100 ml) and washed with water (50 ml) and brine (50 ml). The organic layer was dried over MgSO₄ and concentrated to afford the ketone ester (1.80 g, 71%) as a colorless oil. ¹H NMR (500 MHz, CDCl₃) δ 1.26 (t, J = 6.4 Hz, 3H), 1.44-1.48 (m, 3H), 2.08-2.10 (m, 2H), 2.29-2.31 (d, J = 8.3 Hz, 2H), 2.39-2.40 (m, 4H), 4.18 (q, J = 6.4 Hz, 2H).

**4-Oxocyclohexanecarboxylic acid.** A mixture of ethyl 4-oxocyclohexanecarboxylate (1.74 g, 10 mmol), methanol (25 ml), and 17% aq. KOH (5 ml) was heated at 50 °C for 1.5 h. After being cooled to room temperature, the reaction mixture was acidified to pH 3 with conc. HCl, concentrated to 10 ml under reduced pressure, and extracted with chloroform (3 x 15 ml). The combined organic layers were dried over MgSO₄, filtered, and concentrated to afford the desired ketone acid (1.30 g, 91%) as a colorless solid. mp 62-64 °C; ¹H NMR (500 MHz, CDCl₃) δ 2.05-2.10 (m, 2H), 2.23-2.27 (m, 2H), 2.35-2.41 (m, 2H), 2.49-2.54 (m, 2H), 2.80-2.84 (m, 1H).

**Neopentyl 4-oxocyclohexanecarboxylate.** To a solution of 4-oxocyclohexanecarboxylic acid (852 mg, 6 mmol), triphenylphosphine (1.59 g, 6 mmol), and neopentyl alcohol (635 mg, 7.2 mmol) in dry THF (18 ml) at 0 °C was added dropwise a solution of diethyl azodicarboxylate (0.96 ml, 6 mmol) in dry THF (7.5 ml). The reaction was stirred at rt overnight before being quenched by addition of saturated aqueous NaHCO₃ (50 ml). The aqueous phase was separated and extracted with CH₂Cl₂ (2 x 30 ml). The organic extracts were combined, dried over MgSO₄, and concentrated in vacuo. The residue was diluted with ether (10 ml) and petroleum ether (20 ml) and filtered to remove triphenylphosphine oxide. The solvent was removed in vacuo and the residue was purified by chromatography (20% ether in petroleum ether) to afford the desired ketone ester (820 mg, 65%) as a colorless oil. ¹H NMR (500 MHz, CDCl₃) δ 0.96 (s, 9H), 2.04-2.07 (m, 2H), 2.22-2.25 (m, 2H), 2.34-2.40 (m, 2H), 2.46-2.50 (m, 2H), 2.80 (m, 1H), 3.82 (s, 2H).

**4-Hydroxy-4-(4-fluorophenyl)cyclohexanone ethylene ketal.** To a 500 ml round-bottom flask equipped with a mechanical stirrer, condenser and addition funnel were added magnesium turnings (3.50 g, 140 mmol) and enough THF to cover the Mg. A solution of 1-bromo-4-fluorobenzene (12.45 g, 70.43 mmol) in THF (90 ml) was added dropwise at such a rate that the reaction maintained a gentle reflux following reaction- initiation (the initiation may be accomplished by warming the flask). After the mixture was refluxed for an additional 2.5 h, a solution of 1,4-cylohexanedione monoethylene ketal (10.00 g, 64.03 mmol) in THF (75 ml) was added dropwise. The mixture was kept at refluxing for an additional 2 h before being quenched with saturated ammonium chloride solution (7 ml). After removal of the magnesium salts by filtration, the filtrate was concentrated to dryness. The residue was dissolved in CHCl₃ and washed with water and brine. The organic layer was separated, dried over MgSO₄, and concentrated. The residue was purified by flash chromatography (30% ether in petroleum ether) to afford the desired alcohol (13.50 g, 87%) as a colorless solid. mp 133-134 °C. ¹H NMR (500 MHz, CDCl₃) δ 1.69 (d, J = 11.7 Hz, 2H), 1.79 (d, J = 12.2 Hz, 2H), 2.05-2.18 (m, 4H), 3.98 (m, 4H), 7.02 (t, J = 8.3, 2H), 7.47-7.50 (m, 2H).

**4-Hydroxy-4-(4-fluorophenyl)cyclohexanone.** A mixture of 4-hydroxy-4-(4-fluorophenyl)cyclohexanone ethylene ketal (7.20 g, 28.6 mmol), THF (125 ml) and 5% aq. HCl (65 ml) was refluxed for 14 h. The reaction mixture was cooled to rt, concentrated to 60 ml, and extracted with CH₂Cl₂ (3 x 60 ml). The combined organic layers were dried over MgSO₄ and concentrated in vacuo. The residue was purified by crystallization from hexanes to afford the desired alcohol ketone (5.30 g, 89%) as a colorless solid. mp 111-114 °C (lit. 115-117 °C). ¹H NMR (500 MHz, CDCl₃) δ 2.17-2.20 (m, 2H), 2.23-2.29 (m, 2H), 2.32-2.37 (m, 2H), 2.87-2.94 (m, 2H), 7.04-7.07 (m, 2H), 7.48-7.51 (m, 2H).

**4-Acetoxy-4-(4-fluorophenyl)cyclohexanone.** To a solution of 4-hydroxy-4-(4-fluorophenyl)cyclohexanone (520 mg, 2.5 mmol), pyridine (2 ml) and 4-dimethylaminopyridine (46 mg) in CH₂Cl₂ (25 ml) at 0 °C was added dropwise a solution of acetic anhydride (1 ml) in CH₂Cl₂ (5 ml). The mixture was warmed to room temperature and stirred for 28 h before being quenched with water (30 ml). The organic phase was washed with 1 M HCl (2 x 30 ml) and brine (30 ml), dried over MgSO₄, and concentrated in vacuo. The residue was purified by flash chromatography (25% ether in petroleum ether) to afford the desired ketone (510 mg, 82%) as a colorless solid. mp 113-115 °C. ¹H NMR (500 MHz, CDCl₃) δ 2.11 (s, 3H), 2.20 (m, 2H), 2.43 (m, 2H), 2.68 (m, 2H), 2.86 (m, 2H), 7.05 (t, J = 8.3, 2H), 7.35-7.38 (m, 2H).

**General procedure for the preparation of 1,2,4-trioxolanes.** Ozone was produced with an OREC ozone generator (0.6 L/min O₂, 60 V), passed through an empty gas washing bottle that was cooled to -78 °C, and bubbled through a solution of an *O-*methyl ketone oxime and a ketone in pentane/CH₂Cl₂ at 0 °C. *O*-methyl oximes of cyclohexanone, 2-adamantanone, and 3,3,5,5-tetramethylcyclohexanone (1 mmol) were consumed within 3 min while *O*-methyl cyclododecanone oxime (1 mmol) required 6 min to disappear. After completion, the solution was flushed with oxygen for 5 min before being concentrated in vacuo at room temperature to give a residue that was purified by flash chromatography.

### EXAMPLE 2

### Antimalarial Activity of OZ01-OZ270

Each trioxolane was screened against the chloroquine-resistant K1 and chloroquine-sensitive NF54 strains of *P. falciparum in vitro.* In the single dose STI *in vivo* screen, Moro SPF or NMRI mice infected with the ANKA strain of *P. berghei* (groups of three mice) were treated on day one post-infection with trioxolanes dissolved or suspended in 3% ethanol and 7% Tween 80. Trioxolanes were administered as single 10 mg/kg doses sc and po. Trioxolanes were also administered as single 10 mg/kg doses in standard suspending vehicle (SSV). SSV consists of 0.5% w/v CMC, 0.5% v/v benzyl alcohol, 0.4% v/v Tween 80, and 0.9% w/v sodium chloride in water. Antimalarial activity was measured by percent reduction in parasitemia on day three post-infection and survival times compared to an untreated control group. Survival to day 30 post-infection is considered to be a cure. For comparative analysis, in Table 1 below data is presented for trioxolanes OZ01-OZ270 along with the controls, fenozan, artemisinin, arteether, artemether, and artesunate:

**Table 1**

| Compd | IC₅₀ (ng/ml) K1/NF54 | Activity (%) po/SSVpo/sc | Survival (days) po/SSVpo/sc |
|---|---|---|---|
| NONE | --- | 0 | 5.6 ± 0.3 |
| OZO1 | >100/>100 | 0/NA/0¹ | 5.3/NA/5.3¹ |
| OZ02 | >100/94 | 0/NA/0¹ | 5.0/NA/5.0¹ |
| OZ03 | 1.2/1.6 | 94/NA/100 | 7.2/NA/11.9 |
| OZ04 | >100/>100 | 0/NA/0¹ | 5.3/NA/5.3¹ |
| OZ05 | 0.19/0.36 | 94/74/99.7 | 7.1/6.2/8.6 |
| OZ06 | 18/22 | T/NA/T¹ | 1.0/NA/1.0¹ |
| OZ07 | 66/49 | 0/NA/0¹ | 6.0/NA/5.7¹ |
| OZ08 | 2.4/2.8 | 99.7/NA/99.9¹ | 8.0/NA/9.7¹ |
| OZ09 | 32/39 | 0/NA/81¹ | 5.0/NA/6.3¹ |
| OZ10 | 0.40/0.67 | 99.6/NA/99.98 | 8.9/NA/10.5 |
| OZ11 | 1.5/2.7 | 99.99/98/100 | 11.4/7.4/18 |
| OZ12 | 0.60/0.77 | 75/NA/99.8 | 7.0/NA/9.3 |
| OZ13 | 0.40/0.50 | 56/NA/99.7 | 6.7/NA/9.7 |
| OZ14 | 91/>100 | 23/NA/96 | 6.0/NA/7.7 |
| OZ15 | 0.60/1.0 | 99.8/88/99.9 | 7.4/6.8/8.2 |
| OZ16 | 0.45/0.67 | 79/83/99.2 | 6.7/6.7/7.3 |
| OZ17 | 85/>100 | 0/NA/18 | 5.3/NA/6.3 |
| OZ18 | 63/84 | 0/NA/0 | 5.0/NA/5.3 |
| OZ19 | 1.3/2.7 | 98.3/56/99.98 | 7.5/6.0/13.4 |
| OZ20 | 15/20 | 97/14/90 | 7.4/6.0/7.0 |
| OZ21 | 14/25 | 0/NA/0 | 5.0/NA/6.0 |
| OZ22 | 0.32/0.56 | 35/NA/99.6 | 6.0/NA/7.7 |
| OZ23 | 0.29/0.57 | 99.5/99.34/99.9 | 7.7/7.0/8.9 |
| OZ24 | 0.35/0.58 | 93/66/99.9 | 7.0/6.8/8.7 |
| OZ25 | 1.1/2.1 | 99.9/96/99.97 | 8.4/7.2/16.5 |
| OZ26 | 3.1/5.0 | 88/NA/99.9 | 7.0/NA/9.7 |
| OZ27 | 2.2/4.8 | 99.97/96/99.92 | 12.6/8.0/17.1 |
| OZ28 | 2.1/2.5 | 93/NA/99.97 | 7.3/NA/12.3 |
| OZ29 | 9.7/13 | 0/NA/0 | 5.0/NA/5.0 |
| OZ30 | 0.65/1.3 | 0/NA/86 | 5.3/NA/9.0 |
| OZ31 | 3.0/3.0 | 99.95/90/99.95 | 9.0/7.3/17.7 |
| OZ32 | 0.25/0.51 | 79/93/91 | 6.7/7.0/8.7 |
| OZ33 | 2.3/3.5 | 80/NA/99.97 | 6.7/NA/11.7 |
| OZ34 | 42/60 | 0/NA/0 | 5.3/NA/5.7 |
| OZ35 | 1.7/2.1 | 74/NA/99.98 | 6.3/NA/9.3 |
| OZ36 | 24/62 | 0/NA/0 | 5.7/NA/5.7 |
| OZ37 | 1.1/1.6 | 83/NA/99.98 | 6.7/NA/13 |
| OZ38 | 0.86/1.3 | 83/15/99.98 | 6.7/5.0/25.3 |
| OZ39 | 1.4/1.8 | 61/NA/99.3 | 6.3/NA/7.7 |
| OZ40 | 1.4/2.0 | 99/NA/99.8 | 7.3/NA/9.0 |
| OZ41 | 3.5/4.6 | 99.98/81/99.5 | 12.1/6.8/10.7 |
| OZ42 | 2.3/2.6 | 68/NA/92 | 6.0/NA/7.0 |
| OZ43 | 11/7.5 | 99.97/18/97 | 8.9/5.4/7.3 |
| OZ44 | 2.5/2.3 | 99.3/NA/99.9 | 7.0/NA/8.3 |
| OZ45 | 17/27 | 15/NA/16 | 6.0/NA/5.0 |
| OZ46 | 22/19 | 99/NA/62 | 7.7/NA/6.0 |
| OZ47 | 7.5/7.4 | 99.98/NA/99 | 12.7/NA/8.0 |
| OZ48 | 11/13 | 99.8/NA/42 | 8.7/NA/6.3 |
| OZ49 | 0.82/0.93 | 81/NA/85 | 6.7/NA/7.3 |
| OZ50 | 1.1/1.2 | 77/57/97 | 7.0/5.7/7.3 |
| OZ51 | 2.5/2.5 | 89/NA/100 | 7.0/NA/14 |
| OZ52 | 3.1/3.0 | 88/NA/99.7 | 6.7/NA/8.0 |
| OZ53 | 1.4/1.7 | 9/NA/58 | 6.0/NA/6.3 |
| OZ54 | 32/35 | 85/NA/97 | 7.0/NA/7.0 |
| OZ55 | 0.49/0.60 | 45/NA/97 | 6.0/NA/7.3 |
| OZ56 | 0.59/0.64 | 93/63/99.97 | 6.7/5.7/10.3 |
| OZ57 | 3.1/2.8 | 32/NA/98.4 | 6.0/NA/7.7 |
| OZ58 | 7.1/7.2 | 39/NA/52 | 6.0/NA/6.3 |
| OZ59 | 1.4/1.3 | 51/NA/99.95 | 6.0/NA/10.0 |
| OZ60 | 1.1/1.2 | 71/NA/100 | 6.0/NA/10.3 |
| OZ61 | 0.52/0.53 | 91/NA/96 | .7.0/NA/7.3 |
| OZ62 | 33/28 | 0/NA/0 | 6.0/NA/5.0 |
| OZ63 | 1.6/1.6 | 99.62/65/99.91 | 7.3/6.0/9.3 |
| OZ64 | 1.5/1.3 | 95/NA/98 | 7.3/NA/8.0 |
| OZ65 | 57/65 | 0/NA/1 | 5.0/NA/5.0 |
| OZ66 | 44/36 | 0/NA/0 | 5.3/NA/5.7 |
| OZ67 | >100/>100 | 0/NA/0 | 5.0/NA/5.0 |
| OZ68 | 41/49 | 1/NA/6 | 5.0/NA/5.3 |
| OZ69 | 1.6/1.4 | 56/NA/40 | 6.3/NA/6.3 |
| OZ70 | 1.2/1.1 | 98/NA/94 | 7.7/NA/7.7 |
| OZ71 | 4.5/3.7 | 98/98/97 | 7.3/6.7/8.3 |
| OZ72 | 15/13 | 97/NA/95 | 7.7/NA/6.7 |
| OZ73 | 6.5/8.2 | 27/NA/36 | 6.0/NA/6.7 |
| OZ74 | 2.3/3.5 | 63/NA/99.97 | 6.3/NA/10.0 |
| OZ75 | 2.4/4.3 | 77/NA/5 | 7.3/NA/5.0 |
| OZ76 | 1.7/3.0 | 49/NA/94 | 6.3/NA/8.0 |
| OZ77 | 1.4/2.2 | 99.9/74/98 | 9.0/6.0/8.3 |
| OZ78 | 24/42 | 94/97/79 | 8.7/7.0/6.7 |
| OZ79 | 0.38/0.81 | 85/99.94 | 7.0/NA/9.3 |
| OZ80 | 0.15/0.34 | 59/NA/84 | 6.3NA//7.7 |
| OZ81 | >100/>100 | 0/NA/0 | 5.3/NA/5.0 |
| OZ82 | 0.48/1.2 | 91/NA/79 | 7.3/NA/7.0 |
| OZ83 | 0.30/0.60 | 92/78/99.8 | 7.0/6.3/8.0 |
| OZ84 | 1.4/2.1 | 92/NA/99.99 | 7.7/NA/14.7 |
| OZ85 | 0.86/1.8 | 34/NA/99.7 | 6.0/NA/8.3 |
| OZ86 | 0.80/2.0 | 77/NA//99.9 | 6.7/NA/11.3 |
| OZ87 | 3.6/4.4 | 22/NA/53 | 5.7/NA/6.0 |
| OZ88 | 5.7/4.2 | 62/NA/99.1 | 6.0/NA/11.7 |
| OZ89 | 0.30/0.75 | 94/89/99.8 | 7.3/6.7/8.7 |
| OZ90 | 0.44/0.84 | 89/95/99 | 7.0/7.0/8.0 |
| OZ91 | 1.4/0.42 | 20/0/85 | 5.7/6.0/7.3 |
| OZ92 | 1.6/0.40 | 81/35/99.98 | 6.7/6.3/10.0 |
| OZ93 | 3.3/0.92 | 57/3/100 | 6.7/6.0/13.3 |
| OZ94 | 57/28 | 21/0/11 | 6.0/5.7/5.3 |
| OZ95 | 2.8/1.3 | 31/0/49 | 6.0/6.0/6.0 |
| OZ96 | 8.4/>10 | 12/14/19 | 5.7/5.7/5.3 |
| OZ97 | 2.2/1.8 | 59/2/66 | 6.7/5.3/7.0 |
| OZ98 | 2.3/0.9 | 72/11/77 | 6.3/5.3/7.3 |
| OZ99 | 77/27 | 30/40/36 | 6.3/5.7/6.3 |
| OZ100 | 1.4/0.34 | 61/36/80 | 6.7/6.3/7.0 |
| OZ101 | 3.0/1.6 | 44/0/99.97 | 6.3/5.3/13 |
| OZ102 | 1.6/0.45 | 92/73/99.97 | 7.0/6.7/19.7 |
| OZ103 | 0.64/0.17 | 86/63/87 | 7.3/6.7/7.3 |
| OZ104 | 1.4/0.50 | 56/0/99.98 | 6.3/5.7/12.0 |
| OZ105 | 5.4/5.0 | 16/0/28 | 5.7/5.7/6.3 |
| OZ106 | 2.2/1.7 | 0/0/0 | 5.3/5.05.3 |
| OZ107 | 1.0/0.30 | 70/32/99.74 | 6.3/6.3/8.0 |
| OZ108 | 68/29 | 0/0/0 | 5.0/5.0/5.7 |
| OZ109 | 21/24 | 2/0/24 | 5.7/5.7/6.3 |
| OZ110 | 5.3/2.1 | 50/0/97.97 | 6.7/5.3/7.7 |
| OZ111 | 0.92/0.35 | 98/79/99.94 | 7.7/6.3/8.3 |
| OZ112 | >10/>10 | 6/0/36 | 5.7/5.7/6.3 |
| OZ113 | 0.95/0.20 | 92/96/89 | 7.3/8.0/7.3 |
| OZ114 | 3.7/2.2 | 0/0/99.64 | 5.7/6.0/8.7 |
| OZ115 | 11/6.9 | 33/0/97 | 6.3/5.7/7.3 |
| OZ116 | 4.2/3.3 | 97/96/96 | 7.7/8.3/8.0 |
| OZ117 | 2.1/1.2 | 95/96/99.94 | 7.0/7.7/8.7 |
| OZ118 | 1.0/0.24 | 99.0/98/99.57 | 7.0/8.0/8.3 |
| OZ119 | 0.83/0.20 | 99.29/99.15/99.66 | 7.7/8.0/8.3 |
| OZ120 | 1.2/0.59 | 33/8/96 | 6.0/5.7/8.0 |
| OZ121 | 0.96/0.41 | 91/98/99.61 | 7.3/8.0/7.3 |
| OZ122 | >100/>100 | 10/3/0 | 5.7/5.3/5.0 |
| OZ123 | 1.6/1.9 | 99.66/94/99.96 | 8.0/7.0/16 |
| OZ124 | 2.1/1.7 | 72/17/86 | 6.3/5.3/7.0 |
| OZ125 | 68/>100 | 8/0/0 | 5.3/5.7/6.0 |
| OZ126 | 0.20/0.50 | 55/72/99.44 | 7.0/6.7/8.5 |
| OZ127 | 0.61/1.3 | 95/98/97 | 7.0/7.7/7.7 |
| OZ128 | 0.59/1.1 | 99.93/99.95/99.98 | 8.3/8.7/11.7 |
| OZ129 | 10/>10 | 0/4/0 | 5.3/5.7/5.7 |
| OZ130 | 0.62/0.94 | 98.8/98.9/98.6 | 9.0/8.0/7.3 |
| OZ131 | 1.7/4.1 | 21/41/90 | 5.7/6.0/8.0 |
| OZ132 | 9.8/>10 | 38/0/40 | 6.3/5.7/6.3 |
| OZ133 | 0.70/1.1 | 94/97/72 | 7.0/7.3/7.0 |
| OZ134 | 0.88/1.1 | 72/27/99.95 | 6.3/6.0/15.3 |
| OZ135 | >100/>100 | 1/0/0 | 5.3/5.3/5.3 |
| OZ136 | 23/21 | 0/0/0 | 6.0/6.0/6.0 |
| OZ137 | 11/18 | 0/6/61 | 5.7/6.3/7.0 |
| OZ138 | 10/19 | 0/5/7 | 5.7/6.3/6.3 |
| OZ140 | 5.9/8.2 | 0/8/60 | 6.0/6.3/7.0 |
| OZ141 | 1.7/1.9 | 98/97/99.89 | 8.0/8.0/8.7 |
| OZ142 | 2.3/2.3 | 0/2/99.98 | 6.0/6.3/14.3 |
| OZ143 | 0.98/1.8 | 48/44/99.85 | 6.7/6.7/9.7 |
| OZ144 | 1.4/2.1 | 99.45/92/99.94 | 7.3/7.3/16.7 |
| OZ145 | 0.50/0.76 | 99.82/99.21/99.87 | 8.3/10.7/10.7 |
| OZ146 | 2.2/2.7 | 62/40/91 | 7.0/6.3/9.0 |
| OZ147 | 1.1/1.8 | 85/71/99.89 | 8.0/8.0/19.7 |
| OZ148 | 17/16 | 38/58/21 | 7.3/7.7/6.3 |
| OZ149 | 8.0/8.8 | 64/3/87 | 7.7/6.0/14.3 |
| OZ151 | 3.8/4.0 | 71/71/99.63 | 7.3/7.3/11.0 |
| OZ152 | 3.2/7.2 | 0/12/22 | 6.3/6.7/6.7 |
| OZ153 | 7.7/19 | 15/23/20 | 6.3/7.0/6.3 |
| OZ154 | 5.0/6.1 | 99.74/81/59 | 14.7/8.7/7.7 |
| OZ155 | 12/>10 | 53/53/73 | 6.3/7.0/8.3 |
| OZ156 | 2.1/2.1 | 99.98/98.8/99.98 | 17.0/10.3/19.7 |
| OZ157 | 0.20/0.22 | 90/80/97 | 7.3/8.3/8.3 |
| OZ159 | 0.70/0.94 | 34/31/98 | 6.7/6.7/9.0 |
| OZ160 | 0.70/0.87 | 45/43/99.94 | 6.3/6.7/12.0 |
| OZ161 | 0.40/0.50 | 59/46/99.96 | 7.0/6.7/12.7 |
| OZ162 | 0.50/0.71 | 41/22/99.55 | 6.3/6.3/9.3 |
| OZ163 | 0.2/0.2 | 99.90/99.94/100 | 8.0/9.0/9.7 |
| OZ164 | 59/>10 | 11/0/7 | 5.7/5.7/6.3 |
| OZ165 | 39/>10 | 18/3/9 | 6.3/6.0/5.7 |
| OZ166 | 28/>10 | 11/0/4 | 6.7/6.3/5.7 |
| OZ167 | 6.7/>10 | 0/6/97 | 6.0/6.0/7.3 |
| OZ169 | 15/>10 | 98/22/99.76 | 7.7/6.7/9.0 |
| OZ171 | 1.3/1.4 | 98/85/99.94 | 9.0/7.0/9.7 |
| OZ172 | 3.5/5.0 | 68/82/100 | 8.0/10.3/10.3 |
| OZ173 | 58/32 | 15/0/80 | 7.0/6.7/9.7 |
| OZ174 | 27/34 | 0/15/90 | 6.7/7.3/10.3 |
| OZ175 | 1.4/2.0 | 99.1/97/92 | 10.0/12.3/8.7 |
| OZ176 | 25/35 | 11/18/24 | 7.0/7.7/7.0 |
| OZ177 | 0.9/1.7 | 99.91/99.88/99.91 | 12.3/12.7/17.7 |
| OZ178 | 21/27 | 32/23/31 | 7.3/7.3/10.0 |
| OZ179 | 1.3/1.1 | 99.91/99.78/99.91 | 11.3/10.3/11.3 |
| OZ180 | 3.7/2.8 | 99.91/97/65 | 13.7/12.7/10.3 |
| OZ181 | 0.58/0.35 | 99.98/99.91/100 | 9.0/10.0/11.0 |
| OZ182 | 4.5/5.5 | 91/96/95 | 8.0/7.3/7.7 |
| OZ183 | 0.80/2.1 | 65/19/81 | 6.0/6.0/8.3 |
| OZ184 | 1.0/1.4 | 54/59/97 | 6.7/7.0/8.7 |
| OZ185 | 1.1/1.4 | 86/56/99.96 | 7.7/6.7/10.7 |
| OZ186 | >10/>10 | 87/96/67 | 8.0/9.0/8.0 |
| OZ187 | 4.0/6.8 | 77/94/77 | 7.7/10.0/7.0 |
| OZ188 | 1.5/3.0 | 93/98/99.87 | 8.7/10.0/9.0 |
| OZ189 | 1.7/3.0 | 95/99.79/98 | 9.3/9.0/10.7 |
| OZ190 | 0.16/1.0 | 98/78/2 | 8.7/7.7/5.7 |
| OZ191 | 6.0/>10 | 7/5/17 | 5.7/5.7/6.0 |
| OZ192 | 2.5/4.4 | 38/45/99.98 | 6.0/7.0/13.7 |
| OZ193 | 5.5/8.3 | 99.75/93/99.92 | 9.3/8.0/11.0 |
| OZ194 | 2.0/6.6 | 87/73/99.95 | 8.3/8.0/20.3 |
| OZ195 | 2.2/3.7 | 98/99.02/99.75 | 9.3/10.7/9.0 |
| OZ196 | >10/>10 | 10/0/17 | 6.0/5.3/5.7 |
| OZ197 | 1.0/2.0 | 87/90/99.54 | 7.7/8.7/10.3 |
| OZ198 | >10/>10 | 4/6/6 | 5.7/6.0/5.7 |
| OZ199 | 0.69/1.1 | 99.48/76/99.44 | 8.3/7.7/8.7 |
| OZ200 | 1.0/2.9 | 81/78/92 | 7.0/7.3/8.0 |
| OZ201 | 2.0/3.1 | 100/100/100 | 13/10.3/25 |
| OZ202 | 6.0/7.9 | 79/51/74 | 7.0/7.3/7.3 |
| OZ203 | 3.9/6.7 | 87/99.42/99.72 | 7.3/8.7/9.7 |
| OZ204 | >10/>10 | 0/0/0 | 6.0/6.3/6.0 |
| OZ205 | 1.5/2.0 | 99.96/99.94/99.96 | 10.0/8.7/9.7 |
| OZ206 | 1.0/2.7 | 93/92/91 | 8.0/8.7/9.7 |
| OZ207 | 0.33/0.57 | 99.96/99.98/99.98 | 9.3/12.0/11.3 |
| OZ208 | 6.0/6.5 | 0/29/10 | 6.3/6.7/5.7 |
| OZ209 | 0.21/0.32 | 99.94/99.96/99.97 | 9.5/10.0/12.5 |
| OZ210 | 1.4/1.6 | 99.23/77/99.94 | 9.3/8.0/10.3 |
| OZ211 | 1.0/1.2 | 82/78/99.90 | 8.0/7.3/8.7 |
| OZ212 | 2.7/2.9 | 66/25/59 | 6.7/6.7/8.0 |
| OZ213 | 2.3/2.8 | 83/74/85 | 8.0/8.0/8.0 |
| OZ214 | >10/>10 | 44/54/65 | 6.3/7.3/7.7 |
| OZ215 | 6.4/7.3 | 96/25/39 | 9.76.3/7.0 |
| OZ216 | 0.40/0.67 | 62/69/99.02 | 6.3/7.7/11.0 |
| OZ217 | 2.0/2.0 | 67/8/98 | 7.0/6.0/8.0 |
| OZ218 | 2.0/3.0 | 98/99.30/99.68 | 10.0/14.3/9.0 |
| OZ219 | 0.85/1.6 | 99.89/99.82/99.91 | 8.7/8.0/9.3 |
| OZ220 | 6.9/4.9 | 40/0/99.95 | 6.0/5.3/10.0 |
| OZ221 | >10/>10 | 80/87/97 | 6.3/7.0/8.0 |
| OZ222 | 3.9/6.3 | 87/75/99.76 | 7.7/7.0/10.0 |
| OZ223 | 4.0/>10 | 89/79/99.57 | 7.3/6.3/12.0 |
| OZ224 | 2.0/3.0 | 0/5/20 | 6.0/5.7/5.7 |
| OZ225 | 7.3/>10 | 0/0/17 | 5.7/5.7/6.3 |
| OZ226 | 4.0/4.0 | 0/0/99.92 | 5.0/5.3/11.0 |
| OZ227 | 0.20/0.20 | 99.68/99.90/99.84 | 8.3/9.3/10.7 |
| OZ228 | 2.0/2.1 | 99.94/28/99.94 | 10.3/6.0/14.0 |
| OZ229 | 0.24/0.23 | 99.96/99.08/99.98 | 10.0/8.3/12.7 |
| OZ230 | 3.9/3.6 | 0/0/0 | 5.3/5.7/5.7 |
| OZ231 | >10/>10 | 0/0/0 | 5.3/5.7/6.0 |
| OZ232 | 0.30/0.50 | 76/74/99.94 | 8.0/7.3/8.7 |
| OZ233 | 1.7/1.9 | 69/76/99.66 | 7.0/7.7/8.3 |
| OZ234 | 3.0/3.6 | 12/0/0 | 6.0/6.0/6.3 |
| OZ235 | 1.0/2.0 | 99.92/99.97/97 | 8.7/9.0/8.0 |
| OZ236 | 2.0/2.0 | 89/86/98.61 | 6.3/7.7/7.7 |
| OZ237 | 5.7/7.1 | 5/9/52 | 6.0/6.0/6.7 |
| OZ243 | 0.91/1.1 | 87/97/70 | 7.3/8.7/6.3 |
| OZ244 | 4.0/4.2 | 6/0/18 | 5.7/5.3/6.3 |
| OZ247 | 1.8/2.3 | 57/27/99.85 | 6.3/6.0/9.0 |
| OZ251 | 0.60/0.35 | 29/6/99.87 | 6.7/6.0/9.0 |
| OZ252 | 2.3/2.2 | 98.91/99.49/99.82 | 8.3/9.0/9.3 |
| OZ253 | 0.56/0.45 | 75/59/99.82 | 7.0/6.7/9.3 |
| OZ254 | >100/57 | 27/11/2 | 6.0/5.7/5.7 |
| OZ255 | 2.2/1.1 | 99.61/99.54/99.92 | 9.0/8.7/10.0 |
| OZ256 | 0.3/0.2 | 99.70/99.67/99.95 | 8.7/8.7/9.7 |
| OZ257 | 42/21 | 99.00/99.49/99.84 | 8.3/7.7/9.7 |
| OZ258 | 5.8/5.2 | 75/70/99.95 | 6.0/6.0/10.7 |
| OZ260 | 5.6/4.3 | 72/51/81 | 6.7/7.0/7.7 |
| OZ261 | 39/18 | 61/47/92 | 7.0/7.0/8.0 |
| OZ262 | 0.63/0.84 | 96/98/99.39 | 11.7/12.3/10.0 |
| OZ263 | 0.84/1.1 | 99.53/99.45/99.92 | 13.0/13.3/11.0 |
| OZ264 | 1.2/1.5 | 99.61/99.92/99.97 | 11.0/10.3/13.7 |
| OZ265 | 0.56/1.6 | 99/98/99.67 | 13.0/9.7/11.7 |
| OZ266 | 1.1/1.5 | 99/99.39/99.75 | 13.7/11.7/11.7 |
| OZ267 | 0.20/0.34 | 99.92/99.89/99.97 | 8.3/8.7/9.3 |
| OZ268 | 0.44/0.71 | 99.86/99.47/99.92 | 9.3/13.3/9.7 |
| OZ269 | 0.32/0.61 | 98/86/99.92 | 10.7/11.3/9.0 |
| OZ270 | 0.85/1.3 | 99.17/99.47/99.81 | 13.7/12.3/11.3 |
| Fenozan | 3.2/3.0 | 99.95/NA/100 | 8.6/NA/13.4 |
| Artemisinin | 1.2/1.9 | 62/NA/69 | 6.5/NA/6.8 |
| Arteether | 0.41/0.54 | 98/NA/99.99 | 7.8/NA/9.5 |
| Artemether | 0.45/0.36 | 98/99.2/99.78 | 7.9/7.4/9.1 |
| Artesunate | 1.4/1.5 | 65/74/65 | 6.8/6.4/6.8 |

| | | | |
|---|---|---|---|
| ¹data at a single 100 mg/kg dose | | | |

As shown above, antimalarial activity falls off both when the trioxolane peroxide bond is too exposed or is sterically inaccessible to iron(II) species. Other factors influencing antimalarial activity include the stability of carbon radicals formed by β-scission subsequent to the initial electron transfer to the peroxide bond and the influence of steric effects remote from the peroxide bond on the interactions between carbon radicals and potential drug targets. The data also demonstrates that trioxolane carboxylic acids are usually less active than their hydrocarbon, ester, and hydroxamic acid counterparts.

### EXAMPLE 3

### Activity of Trioxolanes Against P. berghei

As shown in Table 2 below, the trioxolane compounds possess impressive antimalarial activity against *P. berghei in vivo* as determined in the 4-day Peters test. Based on po ED₅₀/FD₉₀ values, OZ23 was the most orally active compound of all of the trioxolanes and control compounds.

**Table 2**

| Compd | *P. berghei* | | *P. yoelii* |
|---|---|---|---|
| | ED₅₀/ED₉₀ (mg/kg) | | ED₅₀/ED₉₀ (mg/kg) |
| | po | sc | sc |
| OZ03 | 1.0/2.7 | 0.6/1.1 | 1.6/3.1 |
| OZ05 | 1.5/6.1 | 0.6/1.0 | 1.4/2.5 |
| OZ10 | 1.3/3.0 | 0.5/0.9 | 1/2.2 |
| OZ11 | 1.8/3.1 | 0.5/0.9 | 1.0/2.1 |
| OZ12 | 6.8/10.5 | 0.8/1.4 | 1.1/2.3 |
| OZ15 | 2.9/6.3 | 0.7/1.1 | 1.4/3.6 |
| OZ23 | 0.9/2.0 | 0.5/0.9 | 0.7 |
| OZ25 | 1.2/3.7 | 2.2/3.7 | 2.7/4.3 |
| OZ27 | 1.3/2.6 | 0.2/0.9 | 4.4/10.2 |
| OZ32 | 2.6/5.1 | 0.6/1.3 | 1.2/3.5 |
| Fenozan | 1.9/2.7 | 1.1/2.3 | 1.8/4.4 |
| Artesunate | 2.4/13 | 1.5/6.3 | 5.3/63 |
| Chloroquine | 1.7/3.1 | 1.5/3.0 | 1.6/28.0 |

### EXAMPLE 4

### Neurotoxicity of Trioxolanes

Even though reported clinical neurotoxicity for the semisynthetic artemisinins is very rare (Park et al., 1998), neurotoxicity is a potential drawback for antimalarial peroxides of any structural class. Against the NB2a cell line (Fishwick et al., 1995) trioxolanes OZ03, OZ04, OZ05, OZ07, and OZ08 had relatively high IC₅₀ₛ of 13, 44, 31, 27, and 42 µM, respectively. In this same screen, dihydroartemisinin, the presumed metabolite of all the semisynthetic artemisinins (Titulaer et al., 1991; White, 1994), was quite neurotoxic with an IC₅₀ of 0.22 µM. There was no apparent relationship between trioxolane structure and neurotoxicity and these five trioxolanes.

### EXAMPLE 5

### Onset of Action and Recrudescence of OZ11, OZ27, OZ78, OZ156, OZ175, OZ177, OZ207, and OZ209

### Onset of Action and Recrudescence Experiments

The onset of drug action was determined after a single fixed dose of 100 mg/kg (SSV vehicle) po to groups of five animals on day +3 post- infection (day 0). Parasitemias at this point are usually between 25-40%. The infected controls do not survive beyond day +6 post-infection. The reduction of parasitemia is monitored 12, 24, and 48 h after treatment, and the time of recrudescence (> 5% parasitemia) is assessed by daily blood smears for 14 days, followed by intermittent assessment for up to 60 days.

The onset part of this experiment reveals how rapidly a compound reduces parasite load; the recrudescence part of the experiment provides information about the efficacy of the compound against the parasite. A long delay in recrudescence can be due to a very good antiparasitic effect of the compound or to a compound with a long half-life.

Both the trioxolanes and the artemisinins produced a rapid decline in parasitemia, confirming that they are rapidly acting antimalarial agents. In contrast to both chloroquine and these peroxidic antimalarials, mefloquine has a slow onset of action. Recrudescence (> 5% parasitemia) occurs quite rapidly for artemisinin and artesunate. The time of recrudescence increased for the more lipophilic artemisinin derivatives artemether and arteether.

In contrast to artemether, recrudescence occured much more slowly for the lipophilic trioxolanes OZ11 and OZ27; the recrudescence time for OZ27 was especially marked, superior to that of mefloquine. However, recrudescence times for the relatively polar trioxolanes OZ78 and OZ175 were very similar to that of artemether. The more lipophilic trioxolane (OZ156) of the OZ156/OZ177 pair produced the longest delay in recrudescence, longer than chloroquine, but less than mefloquine. The recrudescence time for OZ177 was roughly equivalent to that of chloroquine.

Strikingly, there was no recrudescence observed for OZ207 and OZ209, two different salt forms (OZ207 - tosylate, OZ209 - mesylate) of aminomethyl trioxolane OZ163 (hydrochloride). The recrudescence data for these two trioxolanes suggests that they are either more powerful antimalarial agents or have longer half-lives than any of the semisynthetic artemisinins.

**Table 3**

| Compd | Time of Recrudescence (days) |
|---|---|
| OZ11 | 22.2 |
| OZ27 | 22.0 (3/5), > 60 (2/5) |
| OZ78 | 11.2 |
| OZ156 | 19.0 (4/5), > 60 (1/5) |
| OZ175 | 13.0 |
| OZ177 | 18.5 |
| OZ207 | > 60 |
| OZ209 | > 60 |
| Artemisinin | 8.4 |
| Artesunate | 8.6 |
| Artemether | 12.0 |
| Arteether | 11.4 |
| Chloroquine | 17.8 |
| Mefloquine | 28.0 |

### EXAMPLE 6

### Treatment of Schistosomiasis

Mice (MORO SPF female 18-20g) were infected with 90 (±10) cecaria of *Schistosoma mansoni* subcutaneously. Following infection, three of the animals were treated with OZ05 p.o., 100 mg/kg on days 7, 14, 21, 28, 35, and 42.

Compared to control mice, two of the treated mice had a reduction in parasitaemia of 100%, and the third mice a reduction in parasitaemia of 53%. In the same assay, mice treated with artemether showed similar activities, but at doses four times higher than that of the OZ05. Further, in the same assay, arteflene (6x600 mg/kg p.o.) and fenozan (6x100 mg/kg p.o.) were inactive.

In addition, the trioxolanes OZ05 200 mg/kg p.o. and OZ11 100 mg/kg p.o. treated once at day 49 of infection showed activity against adult *S. mansoni.* In contrast, artemether shows no activity against adult *S. mansoni.*

### EXAMPLE 7

### Effect of Trioxolanes on Schistosoma Species

### Effect of Trioxolane OZ207 on Schistosoma japonicum

**Table 4**

| Comparative effect of OZ207 and artemether in mice infected with *Schistosoma japonicum* | | | | | | | |
|---|---|---|---|---|---|---|---|
| **Drug** | **Age of worm** | **Dose (mg/kg × 1)** | **Mice without ♀ worm** | **MTWB/ x± SD** | **WRR/ %** | **MFWB/ x± SD** | **FWR R/ %** |
| Control | - | - | 0/8 | 26.6 ± 4.2 | - | 11.6 ± 2.4 | - |
| OZ207 | 35 days | 200 | 4/7 | 9.1 ± 3.9 | 66 | 0.6 ± 0.7 | 95 |
| OZ207 | 35 days | 400 | 4/6 | 4.3 ± 1.2 | 84 | 0.7 ± 1.2 | 94 |
| Artemeth er | 35 days | 400 | 0/7 | 10.1 ± 4.4 | 62 | 3.4 ± 1.6 | 71 |
| OZ207 | 7days | 200 | | 0/8 5.4 ± 2.4 | 81 | 2.1 ± 1.0 | 82 |

| | | | | | | | |
|---|---|---|---|---|---|---|---|
| MTWB, mean total worm burden; WRR, worm reduction rate MFWB, mean female worm burden; FWRR, female worm reduction rate. | | | | | | | |

Table 4 illustrates that the mean total worm burden and mean female worm burden in OZ207 400 mg/kg group was significantly lower than those in artemether 400 mg/kg group (*P*<0.01). The mean female worm burden in OZ207 200 mg/kg group was also significantly lower than that in artemether group (*P*<0.01).

### Effect of Trioxolanes on 21-day-old schistosomules

Mice were infected with 100 *Schistosoma mansoni* cercariae on day 21 post-treatment. Each group was treated per os with trioxolanes at a single dose of 200 mg/kg. Untreated mice served as the control. All groups were killed 4 weeks after treatment and the liver and intestine were removed and separated. The liver and intestine were compressed and alive male and female worms could be seen and counted. The effect of the compounds was evaluated by mean total and female worm burden. The results are shown in Table 5.

### Effect of Trioxolanes on adult schistosomes (49-day-old)

Mice were infected with 100 *Schistosoma mansoni* cercariae on day 49 post-treatment. Each group was treated per os with OZ compounds at a single dose of 400 mg/kg. Untreated mice served as the control. All groups were killed 4 weeks after treatment and the liver and intestine were removed and separated. The liver and intestine were compressed and alive male and female worms could be seen and counted. The effect of the compounds was evaluated by mean total and female worm burden, and the results are set forth in Table 5.

**Table 5**

| *In Vivo* Activity Against *Schistosoma Mansoni* | | |
|---|---|---|
| Compd | % reduction of schistosomule growth at day 21 | % reduction of adult worm growth at day 49 |
| | | |
| OZ 03 | 74 | |
| OZ 05 | 90 | 19, 70¹ |
| OZ 10 | 66 | |
| OZ 11 | 85 | |
| OZ 12 | 78 | |
| OZ 15 | 63 | |
| OZ 16 | 78 | |
| OZ 19 | 77 | |
| OZ 22 | 75 | |
| OZ 23 | 90 | |
| OZ 24 | 65 | |
| OZ 25 | 86 | |
| OZ 27 | 63 | 20 |
| OZ 32 | 73 | 27 |
| OZ 56 | 69 | |
| OZ 71 | 91 | |
| OZ 78 | 82 | |
| OZ 89 | 86 | |
| OZ 90 | 81 | |
| OZ 111 | 71 | |
| OZ 119 | 88 | |
| OZ 145 | 80 | |
| OZ 157 | 65 | |
| OZ 163 | 84 | |
| OZ 205 | 84 | |
| OZ 207 | 93 | |
| Artemether | 82 | |

| | | |
|---|---|---|
| ¹1 x 600 mg per os | | |

### EXAMPLE 8

### Activity of Trioxolanes Against P. berghei

In the single dose ED₅₀/ED₉₀/ED₉₉ determinations, Moro SPF or NMRI mice (group of three) infected with the ANKA strain of *Plasmodium berghei* were treated on day one post-infection. Trioxolanes were dissolved or suspended in the standard suspending vehicle (SSV)* and administered as single 10, 6, 3, 1, 0.3, and 0.1 mg/kg doses po and sc. The SSV consists of 0.5% w/v CMC, 0.5% v/v benzyl alcohol, 0.4% v/v Tween 80, and 0.9% w/v sodium chloride in water. Antimalarial activity was measured by percent reduction in parasitemia on day three post-infection. The ED₅₀/ED₉₀ values were calculated by nonlinear fitting.
*Earlier ED₅₀/ED₉₀ data (Example 3, Table 2) was obtained using 0.5% carboxymethylcellulose or 0.2% Tween 80 vehicle.

**Table 6**

| Compd | ED₅₀ (mg/kg) | ED₉₀(mg/kg) | ED₉₉ (mg/kg) |
|---|---|---|---|
| OZ05 | 8.7 | 12 | 15 |
| OZ11 | 4.4 | 6.2 | 8.2 |
| OZ27 | 2.9 | 5.7 | 9.9 |
| OZ78 | 4.2 | 9.1 | 17 |
| OZ113 | 3.6 | 9.0 | 19 |
| OZ127 | 2.5 | 7.6 | 19 |
| OZ156 | 1.3 | 2.6 | 4.7 |
| OZ175 | 3.5 | 6.2 | 9.9 |
| OZ177 | 2.1 | 3.7 | 5.8 |
| OZ179 | 1.4 | 3.3 | . 6.6 |
| OZ181 | 0.63 | 1.8 | 4.0 |
| OZ205 | 1.6 | 3.3 | 6.0 |
| OZ207 | 0.37 | 1.2 | 3.0 |
| OZ209 | 0.55 | 1.4 | 3.0 |
| OZ219 | 1.6 | 3.0 | 5.2 |
| OZ227 | 2.3 | 4.0 | 6.2 |
| OZ235 | 4.0 | 7.1 | 11 |
| Artesunate | 4.7 | 19 | 60 |
| Artelinate | 4.8 | 10 | 18 |
| Artemether | 2.2 | 4.2 | 7.1 |
| Chloroquine | 1.8 | 3.5 | 5.9 |
| Mefloquine | 4.0 | 5.4 | 6.8 |

Table 6 shows ED50/ED90/ED99 data obtained by po administration of troixolanes in the SSV formulation. The relatively lipophilic artemether is substantially more active than the more polar artesunate and artelinate. A parallel trend is also evident in the trioxolane data. For example, the highly lipophilic OZ156 is more active than its more polar triazole (OZ177, OZ235) and imidazole (OZ179) analogs, although in this case the potency difference is rather small. With one significant exception (OZ181, OZ207, OZ209), the relatively polar trioxolanes OZ78, OZ113, and OZ127 were less active. In summary, trioxolanes OZ177 and OZ179 were as active and OZ156 and OZ181 were more active than chloroquine, the most active control drug.

### EXAMPLE 9

### In Vivo Toxicity Study of OZ23, OZ32, and OZ78

The toxic potential of three lead trioxolanes (OZ23 - trioxolane carbamate, OZ32 - trioxolane alcohol and OZ78 - trioxolane acid) was investigated against artesunate in an exploratory tolerance study in male Wistar rats. The doses administered were 100 or 300 mg/kg/day for OZ23 and OZ32, and 30 or 100 mg/kg/day for OZ78 and artesunate. All compounds were suspended in SSV and administered at a constant volume of 5 mL/kg/day. Control animals received the vehicle (SSV) at a volume of 5 mL/kg/day. Six animals per group were treated for 5 consecutive days and 6 animals per group were kept for an additional 1-week recovery period. Examinations included clinical observations, body weight development, clinical laboratory investigations (hematology, clinical chemistry and urine analysis) at the end of the treatment and recovery periods, respectively. At the end of the scheduled study period the rats were sacrificed and necropsied and selected organs were examined histopathologically. Plasma levels of the trioxolanes and artesunate sodium were analyzed employing validated HPLC/MS assays, and the data examined for evidence of drug accumulation over the course of the study. Results were compared, where possible, to data from previously conducted exploratory pharmacokinetic studies in rats.

All animals survived to the end of the scheduled study period. Clinical observations related to treatment were limited to occasional occurrence of pale feces in animals given the high dose of OZ23, OZ78 or artesunate. Body weight development was reduced during the treatment period for animals receiving the high dose of OZ23 or artesunate, but was mostly compensated during the recovery period. Clinical laboratory investigations revealed minimal and essentially reversible changes mostly in high dose group animals. Liver weights tended to be minimally or slightly increased in animals receiving the trioxolanes or artesunate. Histopathological examinations indicated slight gastric irritation in animals receiving the higher dose of OZ23 or artesunate.

Plasma concentrations of OZ32 and OZ78 observed in the toxicity study in rats were broadly consistent with those noted in exploratory pharmacokinetic studies. Levels of OZ23 were disproportionately higher in the toxicity study than the levels measured in the exploratory pharmacokinetic studies, although insufficient data are available at this stage to confirm the observed non-linearity of OZ23 pharmacokinetics. Importantly, the toxicokinetic analysis revealed no evidence of accumulation of any of the OZ compounds, artesunate sodium or the major metabolite of artesunate sodium, dihydroartemisinin.

In conclusion, the toxicological profile of the trioxolanes was found to be comparable to that of artesunate.

It should be appreciated that the spiro and dispiro 1,2,4-trioxolane compositions of this invention may contain trioxolanes within the scope of the formulas described above, or prodrugs or analogues of these compounds or a racemic mixture of either the D or the L form. Also, minor dosage and formulation modifications of the composition and the ranges expressed herein may be made and still come within the scope and spirit of the present invention.

Having described the invention with reference to particular compositions, theories of effectiveness, and the like, it will be apparent to those of skill in the art that it is not intended that the invention be limited by such illustrative embodiments or mechanisms, and that modifications can be made without departing from the scope or spirit of the invention, as defined by the appended claims. It is intended that all such obvious modifications and variations be included within the scope of the present invention as defined in the appended claims. The claims are meant to cover the claimed components and steps in any sequence which is effective to meet the objectives there intended, unless the context specifically indicates to the contrary.

### CITATIONS

de Almeida Barbosa, L.-C. et al., The Design, Synthesis and Biological Evaluation of Some Stable Ozonides With Anti-malarial Activity. *J*. *Chem. Soc. Perkin Trans. I,* **1996**, 1101-1105.
de Almeida Barbosa, L.-C. et al., Synthesis of Some Stable Oozonides With Anti-malarial Activity. *J. Chem. Soc. Perkin Trans. I,* **1992**, 3251-3252.
Augustine, R.L. Stereochemistry of the catalytic hydrogenation of some bicyclic α,β-unsaturated ketones. J. Org. Chem., 1958, 23, 1853-1856.
Cammenga, H.K. et al., Basic principles of thermoanalytical techniques and their applications in preparative chemistry. Angew. Chem. Int. Ed. Engl. 1995, 34, 1171-1187.
Cumming, J.N. et al., Antimalarial activity of artemisinin (qinghaosu) and related trioxanes: mechanism(s) of action. Adv. Pharmacol. 1997. 37, 254-297.
Dhingra, V.K. et al., Current Status of Artemisinin and Its Derivatives As Antimalarial Drugs. *Life Sci.* **2000,** *66*, 279-300.
Dong, Y.; Vennerstrom, J.L Peroxidic Antimalarials. *Expert Opin. Ther. Patents* **2001,** *11,* 1753-1760.
Fishwick, J., et al., The Toxicity of Artemisinin and Related Compounds on Neuronal and Glial Cells in Culture. Chem.-Biol. Interact. 1995, 96, 263-271.
Griesbaum, K. et al., Diozonides from coozonolyses of suitable *O*-methyl oximes and ketones. Tetrahedron 1997a, 53, 5463-5470.
Griesbaum, K. et al., Ozonolyses of *O*-alkylated ketoximes in the presence of carbonyl groups: a facile access to ozonides. Liebigs Ann./Recueil. 1997b, 1381-1390.
Jefford, C. Peroxidic Antimalarials. Adv. Drug Res. 1997, 29, 271-325.
Kashima, C. et al., Ozonolysis of Five-Membered Heterocycles. *J. Het. Chem.* **1987,** *24,* 637-639.
Meshnick, S.R. et al., Artemisinin and the antimalarial endoperoxides: from herbal remedy to targeted chemotherapy. Microbiol. Rev. 1996, 60, 301-315.
Park, B.K. et al., Safety Assessment of Peroxide Antimalarials: Clinical and Chemical Perspectives. Br. J. Clin. Pharmacol. 1998, 46, 521-529.
Stork, G. et al., The enamine alkylation and acylation of carbonyl compounds. J. Amer. Chem. Soc. 1963, 85, 207-222.
Titulaer, H.A.C., Zuidema, J., and Lugt, C.B. Formulation and pharmacokinetics of artemisinin and its derivatives. Int. J. Pharmaceut. 1991, 69, 83-92.
van Agtmael, M.A. et al., Artemisinin Drugs In the Treatment of Malaria: From Medicinal Herb to Registered Medication. *Trends Pharmacol. Sci.* **1999**, *20*, 199-205.
Vennerstrom, J.L. et al., Synthesis and Antimalarial Activity of Sixteen Dispiro-1,2,4,5-tetraoxane Analogs of WR 148999: Alkyl Substituted 7,8,15,16-Tetraoxadispiro[5.2.5.2]hexadecanes. *J. Med. Chem.* **2000,** *43,* 2753-2758.
Vroman, J.A. et al., Current Progress in the Chemistry, Medicinal Chemistry and Drug Design of Artemisinin Based Antimalarials. *Curr. Pharm. Design* **1999,** *5*, 101-138.
Wesche, D.L. et al., Neurotoxicity of artemisinin analogs in vitro. Antimicrob. Agents. Chemother. 1994, 38, 1813-1819.
White, N.J. Clinical pharmacokinetics and pharmacodynamics of artemisinin and derivatives. Trans. R. Soc. Trop. Med. Hyg. 1994, 88, 41-43.

## Claims

1. A dispiro 1,2,4-trioxolane having the following structure: 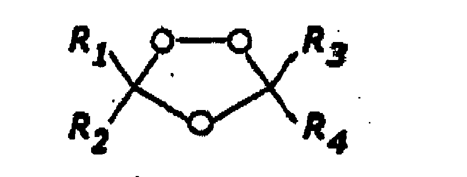 wherein R₁ and R₂ taken together is spiroadamantane and R₃ and R₄ taken together is a spirocyclohexyl ring that is substituted at the 4-position.

2. A dispiro 1,2,4-trioxolane according to claim 1 wherein the spirocyclohexyl ring is interrupted by one or more oxygen, sulfur or nitrogen atoms.

3. A dispiro 1,2,4-trioxolane according to claim 1 wherein the spirocyclohexyl ring is functionalized with a substituted or unsubstituted substituent selected from the group consisting of a linear or branched alkyl, ketone, acid, alcohol, amine, amide, sulfonamide, guanidine, ether, ester, oxime, urea, oxime ether, sulfone, lactone, carbamate, semicarbazone, phenyl, heterocycle and alicyclic group.

4. A dispiro 1,2,4-trioxolane according to claim 1 wherein R3 and R4 are substituted or unsubstituted phenyl or heterocyclic rings.

5. A dispiro 1,2,4-trioxolane according to claim 1 wherein the 1,2,4-trioxolane is selected from the group consisting of: OZ05, OZ11, OZ25, OZ27, OZ61, OZ71, OZ78, OZ127, OZ145, OZ156, OZ163, OZ175, OZ177, OZ179, OZ181, OZ189, OZ205, OZ207, OZ209, OZ210, OZ219, OZ227, OZ229, OZ235, OZ255, OZ256, OZ257, OZ263, OZ264, OZ265, OZ266, OZ267, OZ268, OZ269, and OZ270.

6. A pharmaceutical composition for prophylaxis and treatment of malaria comprising: a malaria prophylaxis or malaria treatment-effective amount of a spiro or dispiro 1,2,4-trioxolane, and optical isomers thereof, and a pharmaceutically acceptable carrier, said trioxolane being sterically hindered on at least one side of the trioxolane heterocycle.

7. A composition according to claim 6, wherein the spiro or dispiro 1,2,4-trioxolane has the following structure: 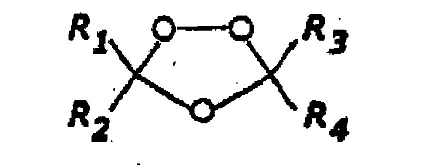 wherein R₁, R₂, R₃, and R₄ are the same or different, and are selected from the group consisting of substituted or unsubstituted linear or branched alkyl, aryl, and alkaryl groups, substituted or unsubstituted alicyclic groups that may be interrupted by one or more oxygen, sulfur or nitrogen atoms, and substituted or unsubstituted aromatic or heterocyclic groups, whereby none of R₁, R₂, R₃, or R₄ may be hydrogen; and further providing that R₁ and R₂ taken together and/or R₃ and R₄ taken together may form a substituted or unsubstituted alicyclic group which is optionally interrupted by one or more oxygen, sulfur or nitrogen atoms.

8. A composition according to claim 7, wherein R₁ and R₂ taken together and/or R₃ and R₄ taken together is a mono- or di-substituted C₅-C₁₂ spirocyclo group which is optionally interrupted by one or more oxygen, sulfur, or nitrogen atoms, said sulfur or nitrogen atoms being optionally substituted.

9. A composition according to claim 8 wherein R₁ and R₂ taken together and/or R₃ and R₄ taken together is spiroadamantane.

10. A composition according to claim 9 wherein R₁ and R₂ taken together is spiroadamantane and R₃ and R₄ taken together is a spirocyclohexyl ring that is substituted at the 4-position.

11. A composition according to claim 9 wherein R₁ and R₂ taken together is spiroadamantane and R₃ and R₄ are substituted or unsubstituted phenyl or heterocyclic rings.

12. A composition according to claim 10 wherein the spirocyclohexyl ring is interrupted by one or more oxygen, sulfur, or nitrogen atoms.

13. A composition according to claim 12 wherein the spirocyclohexyl ring has been replaced with an N-substituted piperidyl.

14. A composition according to claim 9 wherein the spirocyclohexyl ring is functionalized with a substituent selected from the group consisting of a substituted or unsubstituted substituent selected from the group consisting of a linear or branched alkyl, ketone, acid, alcohol, amine, amide, sulfonamide, guanidine, ether, ester, oxime, urea, oxime ether, sulfone, lactone, carbamate, semicarbazone, phenyl, heterocycle, and alicyclic group.

15. A composition according to claim 9 wherein the 1,2,4-trioxolane is selected from the group consisting of: OZ03, OZ05, OZ11, OZ25, OZ27, OZ61, OZ71, OZ78, OZ127, OZ145, OZ156, OZ163, OZ175, OZ177, OZ179, OZ181, OZ189, OZ205, OZ207, OZ209, QZ210, OZ219, OZ227, OZ229, OZ235, OZ255, OZ256, OZ257, OZ263, OZ264, OZ265, OZ266, OZ267, OZ268, OZ269, and OZ270.

16. A method of manufacturing a composition for prophylaxis and treatment of malaria comprising: mixing a malaria prophylaxis or malaria treatment-effective amount of a spiro or dispiro 1,2,4-trioxolane, and optical isomers thereof, with a pharmaceutically acceptable carrier, said trioxolane being sterically hindered on at least one side of the trioxolane heterocycle.

17. A pharmaceutical composition for the treatment of cancer comprising: a cancer treatment-effective amount of a spiro or dispiro 1,2,4-trioxolane, and optical isomers thereof, and a pharmaceutically acceptable carrier, said trioxolane being sterically hindered on at least one side of the trioxolane heterocycle.

18. A pharmaceutical composition for prophylaxis or treatment of schistosomiasis comprising: a schistosomiasis prophylaxis or treatment-effective amount of a spiro or dispiro 1,2,4-trioxolane, and optical isomers thereof, and a pharmaceutically acceptable carrier, said trioxolane being sterically hindered on at least one side of the trioxolane heterocycle.

19. A dispiro 1,2,4-trioxolane having the following structure: 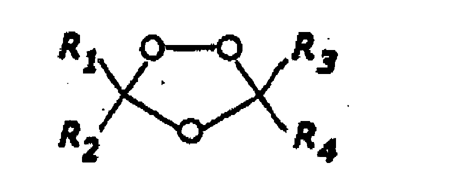 wherein R₁ and R₂ taken together is spiroadamantane and R₃ and R₄ taken together is a spirocyclohexyl ring that is substituted at the 4-position with an alkyl bridge, whereby said spirocyclohexyl ring may be interrupted by one or more oxygen, sulfur, or nitrogen atoms.

20. The dispiro 1,2,4-trioxolane of claim 19 wherein the alkyl bridge is methyl or ethyl.

21. The dispiro 1,2,4-trioxolane of claim 19 wherein the alkyl bridge is functionalized with a substituent selected from the group consisting of a linear or branched alkyl, ketone, acid, alcohol, amine, amide, sulfonamide, guanidine, ether, ester, oxime, urea, oxime ether, sulfone, lactone, carbamate, semicarbazone, phenyl, heterocycle, and alicyclic group.

22. The dispiro 1,2,4-trioxolane of claim 19 wherein the alkyl bridge is functionalized with a substituent that is a weak base.

23. The dispiro 1,2,4-trioxolane of claim 22 wherein the weak base comprises an amide.

24. The dispiro 1,2,4-trioxolane of claim 19 wherein the alkyl bridge is methyl.

25. A method of synthesizing a dispiro 1,2,4-trioxolane comprising: treating a trioxolane having a functional group selected from the group consisting of a ketone, an aldehyde, an ester, and a phthalimide with a reagent to form a compound selected from the group consisting of lactone, alcohol, oxime ether, hydrazone, ketal, acetal, amine, and acid.

## Patentansprüche

1. Dispiro-1,2,4-trioxolan mit der folgenden Struktur: 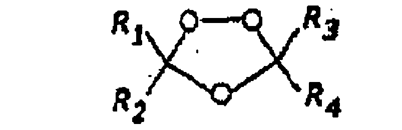 wobei R₁ und R₂ zusammen Spiroadamantan und R₃ und R₄ zusammen ein Spirocyclohexylring sind, der an der 4-Position substituiert ist.

2. Dispiro-1,2,4-trioxolan nach Anspruch 1, wobei der Spirocyclohexylring durch ein oder mehrere Sauerstoff-, Schwefel- oder Stickstoffatome unterbrochen ist.

3. Dispiro-1,2,4-trioxolan nach Anspruch 1, wobei der Spirocyclohexylring mit einem substituierten oder nicht substituierten Substituenten funktionalisiert ist, der ausgewählt ist aus der Gruppe bestehend aus einer linearen oder verzweigten Alkyl-, Keton-, Säure-, Alkohol-, Amin-, Amid-, Sulfonamid-, Guanidin-, Ether-, Ester-, Oxim-, Harnstoff-, Oximether-, Sulfon-, Lacton-, Carbamat-, Semicarbazon-, Phenyl-, Heterozyklus- und alizyklischen Gruppe.

4. Dispiro-1,2,4-trioxolan nach Anspruch 1, wobei R3 und R4 substituierte oder nicht substituierte Phenyl- oder heterozyklische Ringe sind.

5. Dispiro-1,2,4-trioxolan nach Anspruch 1, wobei das 1,2,4-Trioxolan ausgewählt ist aus der Gruppe bestehend aus: OZ05, OZ11, OZ25, OZ27, OZ61, OZ71, OZ78, OZ127, OZ145, OZ156, OZ163, OZ175, OZ177, OZ179, OZ181, OZ189, OZ205, OZ207, OZ209, OZ210, OZ219, OZ227, OZ229, OZ235, OZ255, OZ256, OZ257, OZ263, OZ264, OZ265, OZ266, OZ267, OZ268, OZ269 und OZ270.

6. Pharmazeutische Zusammensetzung zur Vorbeugung gegen und Behandlung von Malaria, die Folgendes umfasst: eine zur Vorbeugung gegen und Behandlung von Malaria wirksame Menge eines Spiro- oder Dispiro-1,2,4-trioxolans (und optische Isomere davon) sowie einen pharmazeutisch akzeptablen Träger, wobei das genannte Trioxolan auf wenigstens einer Seite des Trioxolanheterozyklus sterisch gehindert ist.

7. Zusammensetzung nach Anspruch 6, wobei das Spiro- oder Dispiro-1,2,4-trioxolan die folgende Struktur hat: 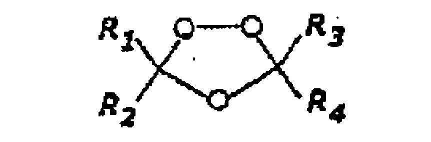 wobei R₁, R₂, R₃ und R₄ gleich oder unterschiedlich sind und ausgewählt sind aus der Gruppe bestehend aus substituierten oder nicht substituierten linearen oder verzweigten Alkyl-, Aryl- und Alkarylgruppen, substituierten oder nicht substituierten alizyklischen Gruppen, die durch ein oder mehrere Sauerstoff-, Schwefel- oder Stickstoffatome unterbrochen sein können, und substituierten oder nicht substituierten aromatischen oder heterozyklischen Gruppen, wobei weder R₁, R₂, R₃ noch R₄ Wasserstoff sein darf; und wobei ferner R₁ und R₂ zusammen und/oder R₃ und R₄ zusammen eine substituierte oder nicht substituierte alizyklische Gruppe bilden können, die optional durch ein oder mehrere Sauerstoff-, Schwefel- oder Stickstoffatome unterbrochen ist.

8. Zusammensetzung nach Anspruch 7, wobei R₁ und R₂ zusammen und/oder R₃ und R₄ zusammen eine mono- oder di-substituierte C₅-C₁₂ Spirocyclogruppe sind, die optional durch ein oder mehrere Sauerstoff-, Schwefel- oder Stickstoffatome unterbrochen ist, wobei die genannten Schwefel- oder Stickstoffatome optional substituiert sind.

9. Zusammensetzung nach Anspruch 8, wobei R₁ und R₂ zusammen und/oder R₃ und R₄ zusammen Spiroadamantan sind.

10. Zusammensetzung nach Anspruch 9, wobei R₁ und R₂ zusammen Spiroadamantan und R₃ und R₄ zusammen ein Spirocyclohexylring sind, der an der 4-Position substituiert ist.

11. Zusammensetzung nach Anspruch 9, wobei R₁ und R₂ zusammen Spiroadamantan und R₃ und R₄ substituierte oder nicht substituierte Phenyl- oder heterozyklische Ringe sind.

12. Zusammensetzung nach Anspruch 10, wobei der Spirocyclohexylring durch ein oder mehrere Sauerstoff-, Schwefel- oder Stickstoffatome unterbrochen ist.

13. Zusammensetzung nach Anspruch 12, wobei der Spirocyclohexylring durch ein N-substituiertes Piperidyl ersetzt wurde.

14. Zusammensetzung nach Anspruch 9, wobei der Spirocyclohexylring mit einem Substituenten funktionalisiert ist, ausgewählt aus der Gruppe bestehend aus einem substituierten oder nicht substituierten Substituenten, ausgewählt aus der Gruppe bestehend aus einer linearen oder verzweigten Alkyl-, Keton-, Säure-, Alkohol-, Amin-, Amid-, Sulfonamid-, Guanidin-, Ether-, Ester-, Oxim-, Harnstoff-, Oximether-, Sulfon-, Lacton-, Carbamat-, Semicarbazon-, Phenyl-, Heterozyklus- und alizyklischen Gruppe.

15. Zusammensetzung nach Anspruch 9, wobei das 1,2,4-Trioxolan auswählt ist aus der Gruppe bestehend aus: OZ03, OZ05, OZ11, OZ25, OZ27, OZ61, OZ71, OZ78, OZ127, OZ145, OZ156, OZ163, OZ175, OZ177, OZ179, OZ181, OZ189, OZ205, OZ207, OZ209, OZ210, OZ219, OZ227, OZ229, OZ235, OZ255, OZ256, OZ257, OZ263, OZ264, OZ265, OZ266, OZ267, OZ268, OZ269 und OZ270.

16. Verfahren zum Herstellen einer Zusammensetzung zur Vorbeugung gegen und Behandlung von Malaria, das die folgenden Schritte umfasst: Vermischen einer zur Vorbeugung gegen oder Behandlung von Malaria wirksamen Menge eines Spiro- oder Dispiro-1,2,4-trioxolans (und optische Isomere davon) mit einem pharmazeutisch akzeptablen Träger, wobei das genannte Trioxolan auf wenigstens einer Seite des Trioxolanheterozyklus sterisch gehindert wird.

17. Pharmazeutische Zusammensetzung zur Behandlung von Krebs, die Folgendes umfasst: eine zur Behandlung von Krebs wirksame Menge eines Spiro- oder Dispiro-1,2,4-trioxolans (und optische Isomere davon) und einen pharmazeutisch akzeptablen Träger, wobei das genannte Trioxolan auf wenigstens einer Seite des Trioxolanheterozyklus sterisch gehindert ist.

18. Pharmazeutische Zusammensetzung zur Vorbeugung gegen oder Behandlung von Schistosomiasis, die Folgendes umfasst: eine zur Vorbeugung gegen oder Behandlung von Schistosomiasis wirksame Menge eines Spiro- oder Dispiro-1,2,4-trioxolans (und optische Isomere davon) und einen pharmazeutisch akzeptablen Träger, wobei das genannte Trioxolan auf wenigstens einer Seite des Trioxolanheterozyklus sterisch gehindert ist.

19. Dispiro-1,2,4-trioxolan mit der folgenden Struktur: 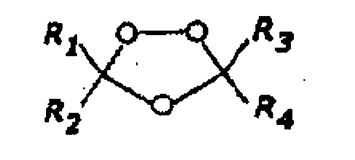 wobei R₁ und R₂ zusammen Spiroadamantan und R₃ und R₄ zusammen ein Spirocyclohexylring sind, der an der 4-Position durch eine Alkylbrücke substituiert ist, wobei der genannte Spirocyclohexylring durch ein oder mehrere Sauerstoff-, Schwefel- oder Stickstoffatome unterbrochen sein kann.

20. Dispiro-1,2,4-trioxolan nach Anspruch 19, wobei die Alkylbrücke Methyl oder Ethyl ist.

21. Dispiro-1,2,4-trioxolan nach Anspruch 19, wobei die Alkylbrücke mit einem Substituenten funktionalisiert ist, ausgewählt aus der Gruppe bestehend aus einer linearen oder verzweigten Alkyl-, Keton-, Säure-, Alkohol-, Amin-, Amid-, Sulfonamid-, Guanidin-, Ether-, Ester-, Oxim-, Harnstoff-, Oximether-, Sulfon-, Lacton-, Carbamat-, Semicarbazon-, Phenyl-, Heterozyklus- und alizyklischen Gruppe.

22. Dispiro-1,2,4-trioxolan nach Anspruch 19, wobei die Alkylbrücke mit einem Substituenten funktionalisiert ist, der eine schwache Base ist.

23. Dispiro-1,2,4-trioxolan nach Anspruch 22, wobei die schwache Base ein Amid umfasst.

24. Dispiro-1,2,4-trioxolan nach Anspruch 19, wobei die Alkylbrücke Methyl ist.

25. Verfahren zum Synthetisieren eines Dispiro-1,2,4-trioxolans, das die folgenden Schritte beinhaltet: Behandeln eines Trioxolans, das eine funktionelle Gruppe hat, die ausgewählt ist aus der Gruppe bestehend aus einem Keton, einem Aldehyd, einem Ester und einem Phthalimid, mit einem Reagens, um eine Verbindung zu bilden, ausgewählt aus der Gruppe bestehend aus Lacton, Alkohol, Oximether, Hydrazon, Ketal, Acetal, Amin und Säure.

## Revendications

1. Un dispiro 1,2,4 trioxolane ayant la structure suivante : 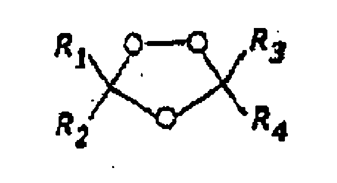 dans laquelle R₁ et R₂ pris collectivement sont un spiroadamantane et R₃ et R₄ pris collectivement sont un cycle spirocyclohexyle substitué en position 4.

2. Un dispiro 1,2,4 trioxolane selon la revendication 1 dans lequel le cycle spirocyclohexyle est interrompu par un ou plusieurs atomes d'oxygène, de soufre ou d'azote.

3. Un dispiro 1,2,4 trioxolane selon la revendication 1 dans lequel le cycle spirocyclohexyle est fonctionnalisé par un substituant substitué ou non substitué sélectionné parmi le groupe consistant en un groupement alkyle, cétone, acide, alcool, amine, amide, sulfamide, guanidine, éther, ester, oxime, urée, éther d'oxime, sulfone, lactone, carbamate, semicarbazone, phényle, hétérocyclique ou alicyclique linéaire ou ramifié.

4. Un dispiro 1,2,4 trioxolane selon la revendication 1 dans lequel R₃ et R₄ sont des cycles phényle ou hétérocycliques substitués ou non substitués.

5. Un dispiro 1,2,4 trioxolane selon la revendication 1 dans lequel le 1,2,4 trioxolane est sélectionné parmi le groupe consistant en : OZ05, 0Z11, OZ25, OZ27, OZ61, OZ71, 0Z78, OZ127, OZ145, OZ156, OZ163, OZ175, OZ177, OZ179, OZ181, OZ189, OZ205, OZ207, OZ209, OZ210, OZ219, OZ227, OZ229, OZ235, OZ255, OZ256, OZ257, OZ263, OZ264, OZ265, OZ266, OZ267, OZ268, OZ269 et OZ270.

6. Une composition pharmaceutique pour la prophylaxie et le traitement du paludisme comprenant : une quantité efficace du point de vue de la prophylaxie et du traitement du paludisme d'un spiro ou dispiro 1,2,4 trioxolane, et d'isomères optiques de celui-ci, et un véhicule pharmaceutiquement acceptable, ledit trioxalane étant stériquement empêché sur au moins un côté de l'hétérocycle trioxolane.

7. Une composition selon la revendication 6, dans laquelle le spiro ou dispiro 1,2,4 trioxolane a la structure suivante : 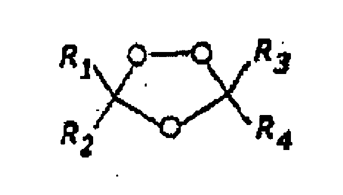 dans laquelle R₁, R₂, R₃ et R₄ sont identiques ou différents, et sont sélectionnés parmi le groupe consistant en des groupements alkyle, aryle et alkylaryle linéaires ou ramifiés substitués ou non substitués, des groupements alicycliques substitués ou non substitués qui peuvent être interrompus par un ou plusieurs atomes d'oxygène, de soufre ou d'azote, et des groupements aromatiques ou hétérocycliques substitués ou non substitués, aucun des radicaux R₁, R₂, R₃ ou R₄ ne pouvant être un hydrogène ; et de plus étant entendu que R₁ et R₂ pris ensemble et/ou R₃ et R₄ pris ensemble peuvent former un groupe alicyclique substitué ou non substitué interrompu optionnellement par un ou plusieurs atomes d'oxygène, de soufre ou d'azote.

8. Une composition selon la revendication 7 dans laquelle R₁ et R₂ pris ensemble et/ou R₃ et R₄ pris ensemble sont un groupe spirocyclo en C₅-C₁₂ mono- ou disubstitué interrompu optionnellement par un ou plusieurs atomes d'oxygène, de soufre ou d'azote, lesdits atomes de soufre ou d'azote étant optionnellement substitués.

9. Une composition selon la revendication 8 dans laquelle R₁ et R₂ pris ensemble et/ou R₃ et R₄ pris ensemble sont un spiroadamantane.

10. Une composition selon la revendication 9 dans laquelle R₁ et R₂ pris ensemble sont un spiroadamantane et R₃ et R₄ pris ensemble sont un cycle spirocyclohexyle substitué en position 4.

11. Une composition selon la revendication 9 dans laquelle R₁ et R₂ pris ensemble sont un spiroadamantane et R₃ et R₄ sont des cycles phényle ou hétérocycliques substitués ou non substitués.

12. Une composition selon la revendication 10 dans laquelle le cycle spirocyclohexyle est interrompu par un ou plusieurs atomes d'oxygène, de soufre ou d'azote.

13. Une composition selon la revendication 12 dans laquelle le cycle spirocyclohexyle a été remplacé par un pipéridinyle N-substitué.

14. Une composition selon la revendication 9 dans laquelle le cycle spirocyclohexyle est fonctionnalisé par un substituant sélectionné parmi le groupe consistant en un substituant substitué ou non substitué sélectionné parmi le groupe consistant en un groupement alkyle, cétone, acide, alcool, amine, amide, sulfamide, guanidine, éther, ester, oxime, urée, éther d'oxime, sulfone, lactone, carbamate, semicarbazone, phényle, hétérocyclique ou alicyclique linéaire ou ramifié.

15. Une composition selon la revendication 9 dans laquelle le 1,2,4 trioxolane est sélectionné parmi le groupe consistant en : OZ05, 0Z11, OZ25, OZ27, OZ61, OZ71, 0Z78, OZ127, OZ145, OZ156, OZ163, OZ175, OZ177, OZ179, OZ181, OZ189, OZ205, OZ207, OZ209, OZ210, OZ219, OZ227, OZ229, OZ235, OZ255, OZ256, OZ257, OZ263, OZ264, OZ265, OZ266, OZ267, OZ268, OZ269 et OZ270.

16. Une méthode de fabrication d'une composition pour la prophylaxie et le traitement du paludisme comprenant : le mélange d'une quantité efficace du point de vue de la prophylaxie ou du traitement du paludisme d'un spiro ou dispiro 1,2,4 trioxolane, et d'isomères optiques de celui-ci, avec un véhicule pharmaceutiquement acceptable, ledit trioxalane étant stériquement empêché sur au moins un côté de l'hétérocycle trioxolane.

17. Une composition pharmaceutique pour le traitement du cancer comprenant : une quantité efficace du point de vue du traitement du cancer d'un spiro ou dispiro 1,2,4 trioxolane, et d'isomères optiques de celui-ci, et un véhicule pharmaceutiquement acceptable, ledit trioxalane étant stériquement empêché sur au moins un côté de l'hétérocycle trioxolane.

18. Une composition pharmaceutique pour la prophylaxie et le traitement de la bilharziose comprenant : une quantité efficace du point de vue de la prophylaxie ou du traitement de la bilharziose d'un spiro ou dispiro 1,2,4 trioxolane, et d'isomères optiques de celui-ci, et un véhicule pharmaceutiquement acceptable, ledit trioxalane étant stériquement empêché sur au moins un côté de l'hétérocycle trioxolane.

19. Un dispiro 1,2,4 trioxolane ayant la structure suivante : 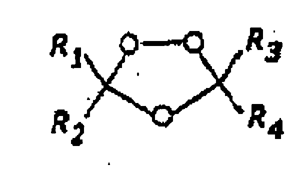 dans laquelle R₁ et R₂ pris collectivement sont du spiroadamantane et R₃ et R₄ pris collectivement sont un cycle spirocyclohexyle substitué en position 4 avec un pont alkyle, ledit cycle spirocyclohexyle pouvant être interrompu par un ou plusieurs atomes d'oxygène, de soufre ou d'azote.

20. Le dispiro 1,2,4 trioxolane de la revendication 19 dans lequel le pont alkyle est un méthyle ou un alkyle.

21. Le dispiro 1,2,4 trioxolane de la revendication 19 dans lequel le pont alkyle est fonctionnalisé par un substituant sélectionné parmi le groupe consistant en un groupement alkyle, cétone, acide, alcool, amine, amide, sulfamide, guanidine, éther, ester, oxime, urée, oxime-éther, sulfone, lactone, carbamate, semicarbazone, phényle, hétérocyclique ou alicyclique linéaire ou ramifié.

22. Le dispiro 1,2,4 trioxolane de la revendication 19 dans lequel le pont alkyle est fonctionnalisé par un substituant qui est une base faible.

23. Le dispiro 1,2,4 trioxolane de la revendication 22 dans lequel la base faible comprend un amide.

24. Le dispiro 1,2,4 trioxolane de la revendication 19 dans lequel le pont alkyle est un méthyle.

25. Une méthode de synthèse d'un dispiro 1,2,4 trioxolane comprenant : le traitement d'un trioxalane ayant un groupement fonctionnel sélectionné parmi le groupe consistant en une cétone, un aldhéhyde, un ester et un phtalimide avec un réactif pour former un composé sélectionné parmi le groupe consistant en une lactone, un alcool, un éther d'oxime, l'hydrazone, un cétal, un acétal, une amine et un acide.
